# EUROPÄISCHE PATENTSCHRIFT

(11) **EP 3 492 595 B1**
(45) Veröffentlichungstag und Bekanntmachung des Hinweises auf die Patenterteilung: **20.12.2023**
(21) Anmeldenummer: 18198740.5
(22) Anmeldetag: 06.06.2014
(51) Int. Cl.: C12N 15/82, C12N 15/113, C07K 14/415

(54) **RESISTENZGEN GEGEN RIZOMANIA**
RESISTANCE AGAINST RHIZOMANIA
GÈNE DE RESISTANCE CONTRE LA RHIZOMANIE

(30) Priorität: 17.06.2013 DE 102013010026
(43) Veröffentlichungstag der Anmeldung: 05.06.2019
(62) Teilanmeldung aus: 14750139.9
(73) Patentinhaber: KWS SAAT SE & Co. KGaA, 37574 Einbeck (DE)
(72) Erfinder: TÖRJÉK, Ottó, 37574 Einbeck (DE); BORCHARDT, Dietrich, 37574 Einbeck (DE); MECHELKE, Wolfgang, 37574 Einbeck (DE); LEIN, Jens Christoph, 37085 Göttingen (DE)
(74) Vertreter: Eisenführ Speiser

(56) Entgegenhaltungen:
- GIDNER SARA ET AL: "QTL mapping of BNYVV resistance from the WB41 source in sugar beet", GENOME, Bd. 48cl, Nr. 2, April 2005 (2005-04), Seiten 279-285, XP009180707, ISSN: 0831-2796
- GRIMMER M K ET AL: "An anchored linkage map for sugar beet based on AFLP, SNP and RAPD markers and QTL mapping of a new source of resistance to Beet necrotic yellow vein virus", THEORETICAL AND APPLIED GENETICS ; INTERNATIONAL JOURNAL OF PLANT BREEDING RESEARCH, SPRINGER, BERLIN, DE, Bd. 114, Nr. 7, 9. Februar 2007 (2007-02-09), Seiten 1151-1160, XP019510491, ISSN: 1432-2242, DOI: 10.1007/S00122-007-0507-3
- LEIN JENS CHRISTOPH ET AL: "Resistance gene analogues are clustered on chromosome 3 of sugar beet and cosegregate with QTL for rhizomania resistance", GENOME, Bd. 50, Nr. 1, Januar 2007 (2007-01), Seiten 61-71, XP001526559, ISSN: 0831-2796
- TIAN YANYAN ET AL: "The absence of TIR-type resistance gene analogues in the sugar beet (Beta vulgaris L.) genome.", JOURNAL OF MOLECULAR EVOLUTION, Bd. 58, Nr. 1, Januar 2004 (2004-01), Seiten 40-53, XP002731101, ISSN: 0022-2844
- BUTORINA A K ET AL: "Molecular genetic investigation of sugar beet (L.)", RUSSIAN JOURNAL OF GENETICS, NAUKA/INTERPERIODICA, MO, Bd. 47, Nr. 10, 8. Oktober 2011 (2011-10-08), Seiten 1141-1150, XP019962364, ISSN: 1608-3369, DOI: 10.1134/S102279541110005X
- AMIRI R ET AL: "A new RAPD marker for beet necrotic yellow vein virus resistance gene in Beta vulgaris", BIOLOGIA PLANTARUM, KLUWER ACADEMIC PUBLISHERS, DO, Bd. 53, Nr. 1, 21. März 2009 (2009-03-21), Seiten 112-119, XP019670809, ISSN: 1573-8264
- PAVLI OURANIA I ET AL: "Achievements and prospects in breeding for rhizomania resistance in sugar beet", FIELD CROPS RESEARCH, Bd. 122, Nr. 3, Juni 2011 (2011-06), Seiten 165-172, XP002731102, ISSN: 0378-4290

## Beschreibung

### Gebiet der Erfindung

Die vorliegende Erfindung betrifft eine BNYVV-resistente transgene Rote Bete Pflanzenzelle der Art *beta vulgaris* ssp. *vulgaris* var. *conditiva,* umfassend als Transgen ein Nukleinsäuremolekül, das in der Lage ist, eine Resistenz gegenüber einem Pathogen, insbesondere gegenüber "Beet Necrotic Yellow Vein Virus" in einer Pflanze, insbesondere der Gattung Beta, in welcher das Polypeptid exprimiert wird, zu vermitteln. Weiterhin betrifft die Erfindung eine Pflanze oder einen Teil davon, umfassend eine erfindungsgemäße Pflanzenzelle und einen Samen einer solchen Pflanze. Ferner schließt die Erfindung auch ein Verfahren zur Selektion einer BNYVV-resistenten Rote Bete Pflanzenzelle oder Pflanze der Art *beta vulgaris* ssp. *vulgaris* var. *conditiva* ein.

### Hintergrund der Erfindung

Die Rizomania ist die wirtschaftlich bedeutendste Zuckerrübenkrankheit weltweit, die Ertragsverluste von 50 % und mehr verursachen kann. Die Krankheit, die auch "Wurzelbärtigkeit" genannt wird, wird durch das "Beet Necrotic Yellow Vein Virus" (BNYVV) hervorgerufen und durch den bodenbürtigen Protozoen *Polymyxa betae* übertragen. Eine BNYVV-Infektion äußert sich in einer gesteigert Proliferation der dünnen Wurzeln und Seitenwurzeln und in der Ausbildung eines stark verkleinerten Wurzelkörpers mit reduzierten Zuckergehalt. Infizierte Pflanzen zeigen eine verminderte Wasseraufnahme und sind somit empfindlicher gegenüber Trockenstress. Wenn sich die Infektion auf die Gesamtpflanze ausweitet, kommt es zur Gelbfärbung der Blattvenen, zu nekrotischen Läsionen und gelben Flecken auf den Blättern. Da eine kurative Bekämpfung der Krankheit wie bei anderen viralen Krankheiten nicht möglich ist, kann ein Schaden nur über den Anbau von resistenten Sorten verhindert werden. Derzeit sind im Wesentlichen drei Majorgene gegen die Rizomania untersucht: RZ-1 (auch als "Holly" bezeichnet), RZ-2 und RZ-3. Darüber hinaus werden weitere, aber weniger bedeutende Rizomania-Resistenzgene in der Literatur beschrieben. Dabei ist das Resistenzgen RZ-1 bereits in den meisten Zuchtlinien (Saatelter oder/und Pollenspender Elternkomponenten) inkorporiert. Es zeigte sich jedoch, dass in stark infizierten Regionen oder in Regionen mit diversen BNYVV-Pathotypen (bspw. Sohi & Maleki, 2004) die Resistenz, welche durch RZ-1 vermittelt wird, nicht ausreicht. Aus diesem Grund wurde schon vor geraumer Zeit vorgeschlagen RZ-1 mit beispielsweise RZ-2 oder RZ-3 zu kombinieren. RZ-2 und RZ-3 stammen aus *Beta vulgaris* subsp. *maritima*-Quellen (WB42, WB41) und kartieren genetisch in der gleichen Region auf Chromosom 3 des Zuckerrübengenoms, während RZ-1 südlich von RZ-2 und RZ-3 aber ebenfalls auf Chromosom 3 kartiert. Scholten et al. (1999) bestimmte einen Abstand von 20-25 cM zwischen den RZ-Majorgenen RZ-1 und RZ-2. Gidner *et al.* (2005) findet einen kleineren Abstand von 5 cM zwischen RZ-1 und RZ-2 und schließt nicht aus, dass RZ-2 und RZ-3 auf dem gleichen Lokus kartieren. Schmidlin *et al.* (2008) hatte mittels Expressionsanalyse in infizierten Rüben unterschiedlich induzierte Gene identifiziert, welche aber nicht RZ-2 oder RZ-3 entsprachen. In der Studie von Larson *et al.* (2008) wurden mit der MALDI-TOF-MS Methode einige BNYVV induzierte Proteine in der Zuckerrübe detektiert, die Proteine, welche durch RZ-1, RZ-2 oder RZ-3 kodiert werden, konnten die Wissenschaftler jedoch nicht identifizieren. Zudem ist die Sequenzregion, insbesondere um diese Resistenzgene herum, repetitiv, was die Entwicklung von diagnostischen Marker besonders schwierig macht. So sind bis jetzt weder hochauflösende Markerkarten noch verifizierte Kandidatengene für die genannten Rizomania-Resistenzgene öffentlich verfügbar. Außerdem ist bislang der funktionelle Hintergrund dieser RZ-Resistenzgene, d. h. die genetische Struktur, völlig unbekannt.

Für eine nachhaltige Züchtung gegen Rizomania, die der Gefahr von Resistenz-brechenden BNYVV-Isolaten entgegen wirken soll, ist es notwendig, ständig neue Resistenzgene zu identifizieren und diese in die Genpools der Kulturpflanzen wie Zuckerrüben zu integrieren.

### Zusammenfassung der Erfindung

Die vorliegende Erfindung wurde vor dem Hintergrund des vorstehend beschriebenen Stands der Technik gemacht, wobei es Aufgabe der vorliegenden Erfindung ist, ein Nukleinsäuremolekül und/oder ein Polypeptid bereitzustellen, welches in der Lage ist, Resistenz gegenüber Rizomania in einer Pflanze zu vermitteln. Weiterhin ist es Aufgabe eine transgene Rizomania-resistente Pflanze sowie eine Methode zu deren Herstellung bereitzustellen. Weiterhin ist es Aufgabe der vorliegenden Erfindung Methoden zur Nutzung und Entwicklung molekulare Marker bereitzustellen, welche eine effiziente Züchtung gegen Rizomania und die Entwicklung neuer resistenter Pflanzenlinien ermöglichen.

Ausgestaltungen der vorliegenden Erfindung, welche die Aufgabe lösen, basieren auf der genetischen Feinkartierung, Identifizierung, Isolierung und Charakterisierung eines Gens, das aus dem Donor *Beta vulgaris* subsp. *maritima* stammt und für ein Polypeptid bzw. Protein kodiert, das in der Lage ist, Resistenz gegenüber einem Pathogen in einer Pflanze, in welcher das Polypeptid exprimiert wird, zu vermitteln.

Einige der in dieser Anmeldung verwendeten Begriffe werden nachfolgend zunächst näher erläutert:
Der Begriff "etwa" im Zusammenhang mit der Angabe einer Länge einer Nukleotidsequenz bedeutet eine Abweichung um +/- 200 Basenpaaren, bevorzugt um +/- 100 Basenpaaren und besonders bevorzugt um +/- 50 Basenpaaren.

Eine "Pflanze der Gattung Beta" gehört zu der Familie der Fuchsschwanzgewächse (Amaranthaceae). Zu diesen Pflanzen zählen Pflanzen der Spezies *Beta macrocarpa, Beta vulgaris, Beta lomatogona, Beta macrorhiza, Beta corolliflora, Beta trigyna* und *Beta nana.* Eine Pflanze der Spezies *Beta vulgaris* ist insbesondere eine Pflanze der Subspezies *Beta vulgaris* subsp. *maritima* (See-Mangold) oder *Beta vulgaris* subsp. *vulgaris.* Hierzu zählen beispielsweise *Beta vulgaris* subsp. *vulgaris* var. *altissima* (Zuckerrübe i.e.S.), *Beta vulgaris* ssp. *vulgaris* var. *vulgaris* (Mangold), *Beta vulgaris* ssp. *vulgaris* var. *conditiva* (Rote Rübe/Bete), *Beta vulgaris* ssp. *vulgaris* var. *crassa*/*alba* (Futterrübe).

Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d.h. mit diesem Basenpaarungen eingeht. Standardverfahren zur Hybridisierung sind beispielsweise in Sambrook et al. 2001 beschrieben. Vorzugsweise wird darunter verstanden, dass mindestens 60%, weiter bevorzugt mindestens 65%, 70%, 75%, 80% oder 85%, besonders bevorzugt 90%, 91%, 92%, 93%, 94%, 95%, 96%, 97%, 98% oder 99% der Basen des Nukleinsäuremoleküls eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. lonenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 70%, vorzugsweise mindestens 75%, mindestens 80%, mindestens 85%, mindestens 90% oder mindestens 95% Sequenzidentität aufweisen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Der hier verwendete Begriff "stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C. Bevorzugt findet eine Hybridisierung unter stringenten Bedingungen statt.

Unter einem "isolierten Nukleinsäuremolekül" wird ein aus seiner natürlichen bzw. ursprünglichen Umgebung herausgelöstes Nukleinsäuremolekül verstanden. Der Begriff umfasst auch ein synthetisch hergestelltes Nukleinsäuremolekül. Unter einem "isolierten Polypeptid" wird ein aus seiner natürlichen bzw. ursprünglichen Umgebung herausgelöstes Polypeptid verstanden. Der Begriff umfasst auch ein synthetisch hergestelltes Polypeptid.

Ein "molekularer Marker" ist eine Nukleinsäure, die polymorph ist in einer pflanzlichen Population. Dadurch ist ein solcher Marker in der Lage verschiedene allelische Zustände (Allele) zu detektieren und zu unterscheiden. Genutzt werden hierfür bekannte analytische Verfahren wie beispielsweise RFLP, AFLP, SNP, SSR oder KASP. Der Begriff "molekularer Marker" bezieht sich auch auf Nukleotidsequenzen, welche komplementär oder zumindest weitestgehend komplementär oder homolog sind zu genomischen Sequenzen, zum Beispiel Nukleinsäuren welche als Sonden oder Primer eingesetzt werden. Marker, die genetische Polymorphismen beschreiben sind, können unter Verwendung gut etablierter Methoden nachgewiesen werden. Dies sind z.B. eine PCR-basierte Sequenz-spezifische Amplifikation, eine Detektion von ,Restriction Fragment Length Polymorphisms` (RFLP), eine Detektion von Polynukleotid-Polymorphismen mittels ,Allele Specific Hybridization' (ASH), eine Detektion von amplifizierten Variable Sequences' des Pflanzengenoms, eine Detektion einer `Self-Sustained Sequence Replication', eine Detektion von 'Simple Sequence Repeats' (SSRs), eine Detektion von ,Single Nucleotide Polymorphisms` (SNPs), oder eine Detektion von ,Amplified Fragment Length Polymorphisms` (AFLPs). Ferner sind auch die Methoden zur Detektion von 'Expressed Sequence Tags' (ESTs) und SSR-Marker abgeleitet von EST-Sequenzen und ,Randomly Amplified Polymorphic DNA' (RAPD) bekannt.

Ein "Promotor" meint eine nicht-translatierte regulatorische DNA-Sequenz, typischerweise stromaufwärts einer kodierenden Region, welche die Bindestelle für die RNA-Polymerase beinhaltet und die Transkription der DNA initiiert.

Ein "Pathogen" meint einen Organismus, der in Interaktionen mit einer Pflanze zu Krankheitssymptomen an einem oder mehreren Organen bei der Pflanze führt. Zu diesen Pathogenen zählen beispielsweise tierische, pilzliche, bakterielle oder virale Organismen oder Oomyceten.

Unter einer "Pathogeninfektion" ist der früheste Zeitpunkt zu verstehen, zu dem ein Pathogen mit einem pflanzlichen Wirtsgewebe interagiert. Beispielsweise im Fall des viralen Pathogens BNYVV wird dieser durch den Protozoen *Polymyxa betae* übertragen. *Polymyxa* bildet Sporen, die in der Erde über viele Jahrzehnte überdauern können. In diesen Sporen überdauert auch der Virus. Wenn diese Dauersporen zu mobilen Zoosporen auskeimen, kann der Virus über diese in Zellen des pflanzlichen Wirtsgewebes gelangen und dort mit dem Wirt interagieren (Esser 2000).

Pflanzliche "Organe" meinen beispielsweise Blätter, Sprossachse, Stamm, Wurzeln, Hypocotyl, vegetative Knospen, Meristeme, Embryos, Antheren, Ovula oder Früchte. Pflanzliche "Teile" meinen einen Zusammenschluss mehrerer Organe, z.B. eine Blüte oder ein Samen, oder einen Teil eines Organs, z.B. eine Querschnitt durch den Spross. Pflanzliche "Gewebe" sind zum Beispiel Kallusgewebe, Speichergewebe, meristematische Gewebe, Blattgewebe, Sprossgewebe, Wurzelgewebe, Pflanzentumorgewebe oder reproduktives Gewebe. Unter pflanzlichen "Zellen" sind beispielsweise isolierte Zellen mit einer Zellwand oder Aggregate davon oder Protoplasten zu verstehen.

Der Begriff "Resistenz" ist breit zu verstehen und deckt den Bereich des Schutzes von einer Verzögerung bis zur kompletten Hemmung der Entwicklung der Krankheit ab. Ein Beispiel für einen Pathogen von Bedeutung ist der Beet Necrotic Yellow Vein Virus (BNYVV). Vorzugsweise erreicht eine resistente Pflanzenzelle der Erfindung oder resistente Pflanze der Erfindung eine Resistenz gegen BNYVV. Eine Resistenz gegenüber einem Pathogen ist gleichzusetzen mit einer Resistenz gegenüber der Krankheit, welche dieser Pathogen verursacht, beispielsweise ist eine Resistenz gegenüber BNYVV auch eine Resistenz gegenüber Rizomania.

"Transgene Pflanze" bezieht sich auf einen Pflanze, in dessen Genom mindestens eine Nukleinsäure integriert wurde. Dabei kann es sich um eine heterologe Nukleinsäure handeln. Vorzugsweise ist die Nukleinsäure stabil integriert, was bedeutet, dass die integrierte Nukleinsäure in der Pflanze stabil erhalten bleibt, exprimiert werden kann und auch stabil an die Nachkommen vererbt werden kann.

Die vorliegende Erfindung betrifft eine BNYVV-resistente transgene Rote Bete Pflanzenzelle der Art *beta vulgaris* ssp. *vulgaris* var. *conditiva,* umfassend als Transgen ein Nukleinsäuremolekül, das eine Nukleotidsequenz umfasst, welche ausgewählt ist aus
a) einer Nukleotidsequenz, die ein Polypeptid mit einer Aminosäuresequenz gemäß SEQ ID NO: 2 oder SEQ ID NO: 3 kodiert,
b) einer Nukleotidsequenz, die die kodierende Sequenz der DNA-Sequenz gemäß SEQ ID NO: 1 umfasst,
c) einer Nukleotidsequenz, die ein Polypeptid kodiert, welches sich durch Substitution, Deletion und/oder Addition von einer Aminosäure der Aminosäuresequenz, die durch die Nukleotidsequenz nach a) oder b) kodiert wird, von einem Polypeptid, das durch die Nukleotidsequenz nach a) oder b) kodiert wird, ableitet,
d) einer Nukleotidsequenz, die ein Polypeptid kodiert, welches eine Aminosäuresequenz aufweist, die zu mindestens 80% identisch ist zu einer Aminosäuresequenz, welche durch die Nukleotidsequenz nach a) oder b) kodiert wird, oder
e) einer Nukleotidsequenz, welche mindestens die Aminosäurepositionen 168-227 der SEQ ID NO: 2 und mindestens die Aminosäurepositionen 591-613 der SEQ ID NO: 2 und mindestens die Aminosäurepositionen 1013-1072 der SEQ ID NO: 2 oder welche mindestens die Aminosäurepositionen 182-241 der SEQ ID NO: 3, mindestens die Aminosäurepositionen 605-627 der SEQ ID NO: 3 und mindestens die Aminosäurepositionen 1027-1086 der SEQ ID NO: 3 kodiert.

Vorzugsweise vermittelt das Polypeptid, welches durch das Nukleinsäuremolekül kodiert wird, eine Resistenz gegenüber dem viralen Pathogen "Beet Necrotic Yellow Vein Virus" (BNYVV), welcher die Pflanzenkrankheit Rizomania verursacht. Weiterhin vermittelt das Polypeptid, welches durch das Nukleinsäuremolekül kodiert wird, insbesondere in einer Pflanze der Gattung Beta eine Resistenz gegenüber einem Pathogen.

In einer Ausführungsform umfasst das Nukleinsäuremolekül die Nukleotidsequenz nach a). Die Aminosäuresequenz gemäß SEQ ID NO: 2 des kodierten Polypeptids und/oder gemäß SEQ ID NO: 3 des kodierten Polypeptids stellt das Resistenzprotein des RZ-3-Gens dar. Es handelt sich hierbei um ein Resistenzgen/-protein des Typs NBS-LRR, das durch bestimmte Strukturmotive charakterisiert ist. Die allgemeine Struktur von solchen Resistenzproteinen in Pflanzen ist bereits gut untersucht (Martin *et al.* 2003). Jedoch ist das Prinzip der strukturellen Ausgestaltung insbesondere der sog. LRR-Domäne, welche als potentielle Erkennungsdomäne für zumeist unbekannte pathogene Effektoren gilt, nicht vorhersagbar. Demzufolge ist die Identifizierung eines BNYVV-Resistenz-vermittelnden Gens bzw. Proteins allein auf Grundlage der bekannten Strukturmotive unmöglich. Die Identifizierung des RZ-3-Resistenzgens fand im Zuge eines Map-based-Cloning-Prozesses statt, der eine intensive genetische Kartierung und Feinkartierung der Zielregion, in welcher das RZ-3-Resistenzgen zunächst vermutet wurde, erforderte. Die Entwicklungsarbeiten werden weiter unten genauer beschreiben.

Das identifizierte Resistenzprotein gehört zum Typ NBS-LRR und weist eine Nukleotid-bindende Domäne (NBS, auch bekannt unter NB-ARC (nucleotide-binding adaptor shared by APAF-1, R proteins, and CED-4)) entsprechend den Aminosäurepositionen 168-227 der SEQ ID NO: 2 oder entsprechend den Aminosäurepositionen 182-241 der SEQ ID NO: 3, eine Leuzin-reiche Domäne (LRR) entsprechend den Aminosäurepositionen 591-613 der SEQ ID NO: 2 oder entsprechend den Aminosäurepositionen 605-627 der SEQ ID NO: 3 und/oder mindestens eine interne repetitive Domäne (IR; Internal Repeat-Domäne) entsprechend den Aminosäurepositionen 1013-1072 der SEQ ID NO: 2 oder entsprechend den Aminosäurepositionen 1027-1086 der SEQ ID NO: 3 auf. Die NBS-Domäne wird durch die Nukleotide 2019-2882 der SEQ ID NO: 1, die LRR-Domäne durch die Nukleotide 3288-3356 der SEQ ID NO: 1 und die IR-Domäne durch die Nukleotide 4554-4871 der SEQ ID NO: 1 kodiert. Die NB-ARC-Domäne ist eine zentrale Nukleotid-bindende Domäne. Sie ist wahrscheinlich eine funktionelle ATPase-Domäne, welche voraussichtlich die Aktivität eines Resistenzproteins reguliert. Die NB-ARC-Domäne besteht aus drei Subdomänen: NB, ARC1 und ARC2. Charakteristische Motive der NB-ARC-Domäne sind APAF-1 (apoptotic proteaseactivating factor-1), welche mutmaßlich für die hypersensitive Reaktion verantwortlich ist, hhGRExE, Walker-A- oder P-loop, Walker-B, GxP, RNBS-A bis D und MHD (Ooijen et al., 2008). Einige der genannten Motive konnten bereits identifiziert werden. In einer weiteren Ausführungsform umfasst das Nukleinsäuremolekül die Nukleotidsequenz nach b). Die Nukleotidsequenz umfasst die kodierenden Sequenzen der DNA-Sequenz gemäß SEQ ID NO: 1, welche für die Aminosäuresequenzen gemäß SEQ ID NO: 2 und 3 kodieren.

In einer weiteren Ausführungsform umfasst das Nukleinsäuremolekül die Nukleotidsequenz nach c). Diese Nukleotidsequenz kodiert ein Polypeptid, das ein Derivat des Polypeptids darstellt, welches von der Nukleotidsequenz nach a) oder b) kodiert wird. Ein Derivat des Polypeptids stellt eine abgeleitete Aminosäuresequenz dar, welche mindestens eine Substitution, Deletion oder Addition von einer oder mehrerer Aminosäuren aufweist, wobei die Funktionalität des kodierten Polypeptids/Proteins erhalten bleibt. Bei der Substitution einer Aminosäure durch eine andere Aminosäure mit gleichen oder äquivalenten oder ähnlichen chemisch-physikalischen Eigenschaften spricht man von einem "konservativen Austausch" oder "semikonservativen Austausch". Beispiele für physikalisch-chemische Eigenschaften einer Aminosäure sind beispielsweise die Hydrophobie oder die Ladung. Dem Fachmann ist bekannt, welche Aminosäuresubstitution einen konservativen oder semikonservativen Austausch darstellt. Das allgemeine Fachwissen erlaubt es darüber hinaus, dass der Fachmann erkennen, identifizieren und nachweisen kann, welche Aminosäuredeletionen und -additionen für die Funktionalität des Resistenzproteins RZ-3 unschädlich sind und an welchen Positionen diese möglich sind. Dem Fachmann ist bewusst, dass im Falle des vorliegenden NBS-LRR-Proteins für Modifikationen der Aminosäuresequenz (Substitutionen, Deletionen oder Additionen von einer oder mehrerer Aminosäuren) insbesondere die Funktionalität der oben definierten konservierten Domänen erhalten bleiben muss und dass daher in diesen Domänen nur begrenzt vorstehende Modifikationen möglich sind. Die Nukleotidsequenz dieser Ausführungsform kodiert dann für ein Derivat bzw. für eine abgeleitete Aminosäuresequenz, wenn die Nukleotidsequenz zu mindestens 60%, 65%, 70%, 75%, 80%, 85%, 90%, 92%, 94%, 96%, 97%, 98%, 99% homolog oder identisch ist zu der Nukleotidsequenz nach a) oder b) ist. Vorzugsweise können solche Nukleotidsequenzen, welche für ein Derivat bzw. für eine abgeleitete Aminosäuresequenz kodieren, entweder direkt oder indirekt (beispielsweise via Amplifikations- oder Replikationsschritte) aus einer Ausgangsnukleotidsequenz, welche über die Gesamtlänge oder zumindest teilweise der SEQ ID NO: 1 oder einer anderen hier offenbarten Sequenz entspricht, erzeugt werden.

In einer weiteren Ausführungsform umfasst das Nukleinsäuremolekül die Nukleotidsequenz nach d). Diese Nukleotidsequenz kodiert für ein Polypeptid, welches eine Aminosäuresequenz aufweist, die zu mindestens 80%, 85%, 90%, 92%, 94%, 96%, 97%, 98%, 99% identisch ist zu einer Aminosäuresequenz, welche durch die Nukleotidsequenz nach a) oder b) kodiert wird.

In einer weiteren Ausführungsform umfasst das Nukleinsäuremolekül die Nukleotidsequenz nach e). Die Nukleotidsequenz kodiert hierbei mindestens eine Nukleotid-bindende Domäne (NBS) entsprechend den Aminosäurepositionen 168-227 der SEQ ID NO: 2 oder entsprechend den Aminosäurepositionen 182-241 der SEQ ID NO: 3, mindestens eine Leuzin-reiche Domäne (LRR) entsprechend den Aminosäurepositionen 591-613 der SEQ ID NO: 2 oder entsprechend den Aminosäurepositionen 605-627 der SEQ ID NO: 3 und/oder mindestens eine interne repetitive Domäne (IR) entsprechend den Aminosäurepositionen 1013-1072 der SEQ ID NO: 2 oder entsprechend den Aminosäurepositionen 1027-1086 der SEQ ID NO: 3. Bevorzugt kodiert die Nukleotidsequenz für ein Polypeptid, das mindestens eine Nukleotid-bindende Domäne (NBS) entsprechend den Aminosäurepositionen 168-227 der SEQ ID NO: 2 oder entsprechend den Aminosäurepositionen 182-241 der SEQ ID NO: 3, mindestens eine Leuzin-reiche Domäne (LRR) entsprechend den Aminosäurepositionen 591-613 der SEQ ID NO: 2 oder entsprechend den Aminosäurepositionen 605-627 der SEQ ID NO: 3 und mindestens eine interne repetitive Domäne (IR) entsprechend den Aminosäurepositionen 1013-1072 der SEQ ID NO: 2 oder entsprechend den Aminosäurepositionen 1027-1086 der SEQ ID NO: 3 umfasst. Besonders bevorzugt liegen diese Domänen in dem Polypeptid sequentiell vom N- zum C-Terminus in der Reihenfolge NBS - LRR - IR vor, wobei zwischen Domänen jeweils ein oder mehrere weitere Aminosäuren anwesend sein können.

Offenbart ist auch ein Polypeptid, welches in der Lage ist eine Resistenz gegenüber einem Pathogen in einer Pflanze, in welcher das Polypeptid exprimiert wird, zu vermitteln und welches durch das Nukleinsäuremolekül kodiert wird, wobei der Pathogen vorzugsweise BNYVV ist und/oder die Pflanze vorzugsweise eine Pflanze der Gattung Beta, insbesondere eine Pflanze der Spezies *Beta vulgaris,* ist. Besonders bevorzugt weist das Polypeptid eine Aminosäuresequenz gemäß SEQ ID NO: 2 oder gemäß SEQ ID NO: 3 auf. Bei dem Polypeptid kann es sich um ein isoliertes Polypeptid handeln.

Offenbart ist auch ein Vektor, welcher das Nukleinsäuremolekül umfasst. Bei dem Vektor kann es sich um ein Plasmid, ein Cosmid, eine Phage oder einen Expressionsvektor, einen Transformationsvektor, Shuttle-Vektor oder Klonierungsvektor handelt, er kann doppel- oder einzelsträngig, linear oder zirkulär sein oder kann einen prokaryotischen oder eukaryotischen Wirt entweder durch Integration in dessen Genom oder extrachromosomal transformieren. Bevorzugt ist das Nukleinsäuremolekül in einem Expressionsvektor mit einer oder mehreren regulatorischen Sequenzen, welche die Transkription und optional die Expression in einer prokaryotischen oder eukaryotischen Wirtszelle erlauben, operativ verknüpft. Beispielsweise steht das Nukleinsäuremolekül unter der Kontrolle eines geeigneten Promotors oder eines Terminators. Geeignete Promotoren können Promotoren sein, welche konstitutiv induziert werden (Bsp.: 35S-Promoter aus dem "Cauliflower mosaic virus" (Odell et al. 1985), besonders geeignet sind solche Promotoren, welche pathogen-induzierbar sind (Bsp.: PR1-Promotor aus Petersilie (Rushton et al., 1996). Besonders geeignete Pathogen-induzierbare Promotoren sind synthetische bzw. chimäre Promotoren, welche in der Natur nicht vorkommen, aus mehreren Elementen zusammengesetzt sind und einen Minimalpromotor beinhalten sowie stromaufwärts des Minimalpromotors mindestens ein cis-regulatorisches Element aufweisen, welches als Bindungsstelle für spezielle Transkriptionsfaktoren dient. Chimäre Promotoren werden den gewünschten Anforderungen nach konzipiert und werden durch unterschiedliche Faktoren induziert oder reprimiert. Beispiele für solche Promotoren finden sich in WO 00/29592, WO 2007/147395 und WO 2013/091612. Ein geeigneter Terminator ist beispielsweise der nos-Terminator (Depicker et al., 1982).

Zusätzlich zu den oben beschriebenen Vektoren, wird ein Verfahren offenbart, das die Einbringung eines beschriebenen Vektors in eine Wirtszelle umfasst. Der Vektor kann beispielsweise durch Konjugation, Mobilisation, biolistische Transformation, Agrobakterium-vermittelte Transformation, Transfektion, Transduktion, Vakuuminfiltration oder Elektroporation eingebracht werden. Solche Verfahren ebenso wie Verfahren zur Präparation von beschriebenen Vektoren sind dem Fachmann geläufig (Sambrook et al. 2001).

Offenbart ist auch eine Wirtszelle, welche das Nukleinsäuremolekül oder den Vektor umfasst. Eine Wirtszelle im Sinne der Erfindung kann eine prokaryotische (z.B. bakteriell) oder eukaryotische Zelle (z.B. eine pflanzliche Zelle oder eine Hefezelle) sein. Vorzugsweise ist die Wirtszelle ein Agrobakterium wie *Agrobacterium tumefaciens* oder *Agrobacterium rhizogenes* oder eine Pflanzenzelle, welche das Nukleinsäuremolekül oder den Vektor umfassen. Dem Fachmann sind sowohl zahlreiche Methoden wie Konjugation oder Elektroporation bekannt, mit denen er das Nukleinsäuremolekül oder den Vektor in ein Agrobakterium einbringen kann, als auch Methoden wie diverse Transformationsverfahren (biolistische Transformation, Agrobakterium-vermittelte Transformation), mit denen er das Nukleinsäuremolekül oder den Vektor in eine Pflanzenzelle einbringen kann (Sambrook et al. 2001).

Die vorliegende Erfindung betrifft eine transgene Pflanzenzelle, welche das Nukleinsäuremolekül als Transgen umfasst. Eine solche transgene Pflanzenzelle ist beispielsweise eine Pflanzenzelle, welche mit dem Nukleinsäuremolekül, vorzugsweise stabil, transformiert ist. In einer bevorzugten Ausgestaltung der transgenen Pflanzenzelle ist, das Nukleinsäuremolekül mit einer oder mehreren regulatorischen Sequenzen, welche die Transkription und optional die Expression in der Pflanzenzelle erlauben, operativ verknüpft. Das Gesamtkonstrukt aus dem Nukleinsäuremolekül und der/den regulatorischen Sequenzen stellt dann das Transgen dar. Solche regulatorische Sequenzen sind beispielsweise ein Promoter oder ein Terminator. Dem Fachmann sind zahlreiche in Pflanzen anwendbare, funktionelle Promotoren und Terminatoren bekannt. Bevorzugt zeigt eine transgene Pflanzenzelle der vorliegenden Erfindung, insbesondere eine Zelle einer Pflanze der Gattung Beta, gegenüber einem Pathogen, insbesondere BNYVV, eine höhere Resistenz als eine korrespondierende nicht-transformierte Pflanzenzelle (die Pflanzenzelle ohne das Transgen). Das Level der Resistenz beispielsweise gegenüber BNYVV kann in Pflanzen der Gattung Beta qualitativ durch Bestimmung von Boniturnoten festgelegt werden (Boniturnotenschemata für Pflanze der Gattung Beta sind aus dem Stand der Technik bekannt, beispielsweise für Zuckerrüben Mechelke (1997)). Eine höhere Resistenz zeigt sich in einer Verbesserung der Resistenz um mindestens eine Boniturnote, um mindestens zwei Boniturnoten, um mindestens drei oder mehr Boniturnoten. Weiterhin offenbart ist ein Verfahren zur Herstellung einer transgenen Pflanzenzelle der vorliegenden Erfindung, welches einen Schritt des Einbringens des Nukleinsäuremoleküls oder des Vektors in eine pflanzliche Zelle umfasst. Beispielsweise kann das Einbringen durch Transformieren stattfinden, vorzugsweise durch stabiles Transformieren. Die geeigneten Techniken zur Einbringung wie biolistische Transformation, Agrobakterium-vermittelte Transformation oder Elektroporation sind dem Fachmann bekannt (Sambrook et al. 2001).

In einem weiteren Aspekt betrifft die vorliegende Erfindung eine transgene Pflanze oder einen Teil davon, umfassend eine vorstehend beschriebene transgene Pflanzenzelle. Ein Teil kann dabei eine Zelle, ein Gewebe, ein Organ oder ein Zusammenschluss von mehreren Zellen, Geweben, oder Organen sein. Ein Zusammenschluss von mehreren Organen ist z.B. eine Blüte oder ein Samen. In einer besonderen Ausgestaltung betrifft die Erfindung einen Samen von der transgenen Pflanze, wobei der Samen das Nukleinsäuremolekül als Transgen umfasst. Bevorzugt zeigt eine transgene Pflanze der vorliegenden Erfindung, insbesondere eine Pflanze der Gattung Beta, gegenüber einem Pathogen, insbesondere BNYVV, eine höhere Resistenz als eine korrespondierende nicht-transformierte Pflanze (Pflanze ohne das Transgen). Das Level der Resistenz beispielsweise gegenüber BNYVV kann in Pflanzen der Gattung Beta qualitativ durch Bestimmung von Boniturnoten festgelegt werden (Boniturnotenschemata für Pflanze der Gattung Beta sind aus dem Stand der Technik bekannt, beispielsweise für Zuckerrüben Mechelke (1997)). Eine höhere Resistenz zeigt sich in einer Verbesserung der Resistenz um mindestens eine Boniturnote, um mindestens zwei Boniturnoten, um mindestens drei oder mehr Boniturnoten. Ferner offenbart wird ein Verfahren zur Herstellung einer transgenen Pflanze, welches einen Schritt des Einbringens des Nukleinsäuremoleküls oder des Vektors in eine pflanzliche Zelle und optional einen Schritt der Selektion einer transgenen Pflanzenzelle umfasst. Weiterhin ist ein solches Verfahren zur Herstellung einer transgenen Pflanze durch einen anschließenden Schritt gekennzeichnet, der die Regenerierung der transgenen Pflanze aus der im ersten Schritt erzeugten transgenen Pflanzenzelle einschließt. Methoden zur Regeneration sind dem Fachmann aus dem Stand der Technik bekannt.

Weiterhin offenbart ist ein Verfahren zur Vermittlung oder Erhöhung einer Resistenz gegenüber einem Pathogen, insbesondere BNYVV, in einer Pflanze, bevorzugt einer Pflanze der Gattung Beta, welche einen Schritt des Transformierens einer Pflanzenzellen mit dem Nukleinsäuremolekül oder dem Vektor umfasst. Bevorzugt führt dieses Verfahren zu einer Verbesserung der Resistenz um mindestens eine Boniturnote, besonders bevorzugt zu einer Verbesserung der Resistenz um mindestens zwei, drei oder mehr Boniturnoten. Boniturnotenschemata für Pflanze der Gattung Beta sind aus dem Stand der Technik bekannt, beispielsweise für Zuckerrüben Mechelke (1997).

Weiterhin offenbart ist eine regulatorische Sequenz eines Promotors, welche die Expression eines Gens, das das Nukleinsäuremolekül umfasst, kontrolliert, dadurch gekennzeichnet, dass die regulatorische Sequenz in der Lage ist, die Expression einer heterologen DNA-Sequenz infolge einer Pathogeninfektion zu vermitteln oder zu modulieren, und die regulatorische Sequenz ein Nukleinsäuremolekül mit einer Nukleotidsequenz gemäß SEQ ID NO: 1 von den Nukleotiden 1-1403 umfasst. Vorzugsweise ist die heterologe DNA-Sequenz eine Nukleotidsequenz, welche für eine Komponente der pflanzlichen Pathogenabwehr kodiert (Bsp.: Resistenzgene (R-Gene) oder Gene, welche für am Signaltransfer beteiligte Enzyme wie Kinasen oder Phosphatasen sowie für G-Protein kodieren) oder welche für einen pathogenen Effektor kodiert (sog. Avirulenzgene (avr)). Weiterhin offenbart ist ein rekombinantes DNA-Molekül, welches die vorstehend beschriebene regulatorische Sequenz umfasst. Vorzugsweise ist das rekombinante DNA-Molekül mit einer heterologen DNA-Sequenz operativ verknüpft.

Weiterhin offenbart ist eine Wirtszelle, welche mit der vorstehend beschriebenen regulatorischen Sequenz oder mit dem genannten rekombinanten DNA-Molekül transformiert ist sowie auf eine transgene Pflanze, Pflanzengewebe oder Pflanzenzelle, welche die regulatorische Sequenz oder das rekombinante DNA-Molekül als Transgen umfasst. Weiterhin offenbart ist ein Verfahren zur Herstellung einer transgenen Pflanzenzelle, welche einen Schritt des Einbringens der regulatorischen Sequenz der Erfindung oder des rekombinanten DNA-Molekül und optional einen Schritt der Selektion einer transgenen Pflanzenzelle umfasst. Ferner offenbart ist ein Verfahren zur Herstellung einer transgenen Pflanze, welche einen Schritt des Einbringens der regulatorischen Sequenz der Erfindung oder des rekombinanten DNA-Molekül in eine pflanzliche Zelle und optional einen Schritt der Selektion einer transgenen Pflanzenzelle umfasst. Weiterhin ist ein solches Verfahren zur Herstellung einer transgenen Pflanze durch einen anschließenden Schritt gekennzeichnet, der die Regenerierung der transgenen Pflanze aus der im ersten Schritt erzeugten transgenen Pflanzenzelle einschließt.

Wie bereits oben ausgeführt, fand die Identifizierung des RZ-3-Resistenzgens im Zuge eines Map-based-Cloning-Prozesses statt. Der durchgeführte Prozess umfasste beispielsweise die Schritte: Genetische Feinkartierung, physikalische Kartierung, Aufbau einer sehr großen spaltenden Population von über 8000 F2-spaltenden Nachkommen, Rekombinanten-Screen, Markerentwicklung in der Zielregion, vergleichende BAC Sequenzierung in resistenten und sensitiven Genotypen, bioinformatische Analysen, Proteinvorhersagen und Vergleich der Proteine. Solche langwierigen Entwicklungsarbeiten sind immer überaus kostspielig und es ist ungewiss, ob es tatsächlich gelingt das Gen zu identifizieren. Nach Integration des RZ-3-Lokus aus *Beta vulgaris* subsp. *maritima* in einer Pflanze der Gattung Beta, nämlich in Zuckerrübe (*Beta vulgaris* subsp. *vulgaris* var. *altissima)* wurden für die Verfolgung des RZ-3-Genomsegments in der Feinkartierung Marker mit guten diagnostischem Wert entwickelt, was sich als besonders schwierig herausstellte, da die Zielregion über weite Bereiche hin repetitiv ist. Überraschenderweise gelang dennoch die Entwicklung von einigen wenigen diagnostischen Markern, welche teilweise auch nur mit einer bestimmten Markertechnik wie Pyrosequenzen also als PSQ-Marker funktionierten oder nullallelisch waren.

Trotz dieser technischen Schwierigkeiten konnte durch umfangreiche Analyse unter Verwendung dieser Marker eine Eingrenzung des RZ-3-Lokus auf eine genomische Region von 0,67 cM erreicht werden. Dies entspricht einer physikalischen Länge von etwa 340.000 bp. Trotz intensiver Weiterentwicklungen war es nur noch beschränkt möglich, die *Beta vulgaris* subsp. *maritima*-Introgression um das Gen herum Marker-gestützt weiter zu reduzieren und Kandidatengene für das RZ-3-Gen zu identifizieren. Eine weitere Verkürzung der Introgression ist jedoch aus züchterischer Sicht in jedem Fall wünschenswert, um potentiell vorhandenen "Linkage Drag", eng gekoppelt zu dem RZ-3-Gen, zu eliminieren. In mehreren Schritten mittels Feinkartierung und unter Einbindung von Sequenzinformation aus physikalischen Karten konnte schließlich eine Zielregion auf nur noch etwa 0,07 cM eingeengt werden. Dies wurde jedoch nur dadurch möglich, dass insgesamt 8004 Pflanzen untersucht wurden, darunter informative rekombinante BC2S1 oder BC2S2 Pflanzen, welche intensiv mit jeweils 90-180 Nachkommen analysiert wurden. Dieses war notwendig, da die Resistenzausprägung aus unbekannten Gründen nicht immer eindeutig war. Diese Nachkommen wurden einzelpflanzenweise genotypisiert und parallel phänotypisiert. Mittels statistischer Verfahren (t-Test, Poweranalyse) wurden die Phänotypen der informativen Rekombinanten (homozygot resistent - RR; heterozygot resistent - Rs; homozygot anfällig - ss) nachgewiesen und damit Rückschlüsse auf den Genotyp der informativen Rekombinanten gezogen.

In der relativ kleinen Zielregion von etwa 38.000 bp konnten in dem anfälligen Genotyp zehn Gene annotiert werden. Für diese Zielregion wurden aus einer resistenten BAC Bibliothek überlappende Klone mit Hilfe von neuen Markern, welche spezifisch den Zielbereich beschreiben, identifiziert und schließlich sequenziert. Wegen der Repetivität der Zielregion zeigte die Sequenz des anfälligen Genotyps zahlreiche kleine Abschnitte mit unbekanntem Sequenzinhalt. Aus diesem Grund war die Assemblierung der RR- und ss-Sequenzen besonders anspruchsvoll. Es konnte dennoch ein putatives Resistenzgen identifiziert werden. Dieses enthielt in nahezu allen ss-Genotypen ein Retrotransposon mit einer Länge von ca. 8000 bp zwischen der LRR-Domäne und der IR-Domäne, was in RR-Genotypen nicht detektiert werden konnte. Eine Aminosäuresequenz, vorhergesagt aus der putativen Resistenzgensequenz, zeigt, dass das Gen mutmaßlich für ein NB-ARC-LRR-Protein kodiert. Es kann vermutet werden, dass diese Insertion des Retrotransposons die Funktion des Gens in anfälligen ss-Genotypen zerstört, denn sie trennt die Internal Repeat-Domäne (IR) von den beiden anderen Domänen (NB-ARC und LRR).

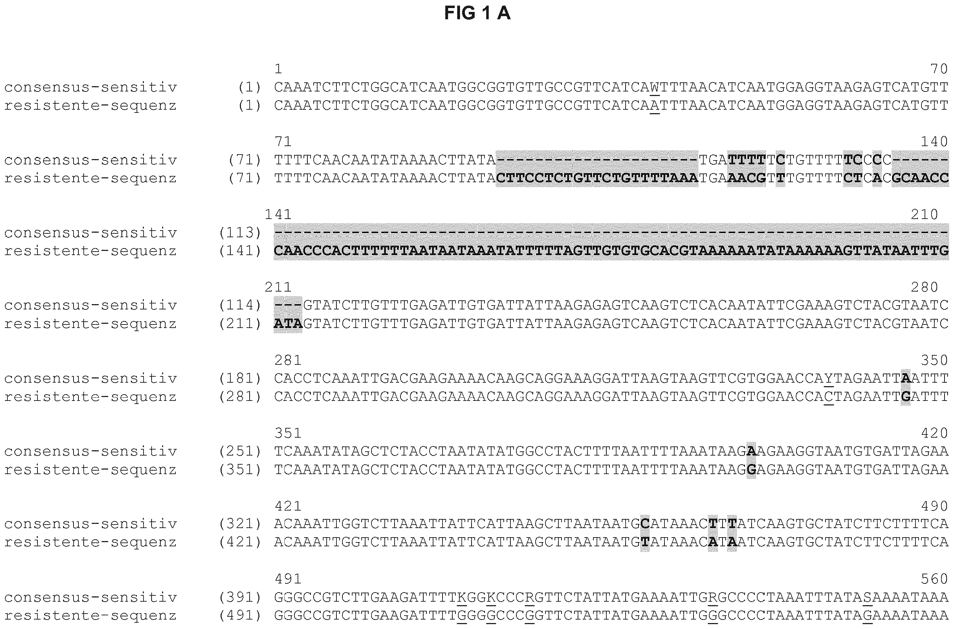

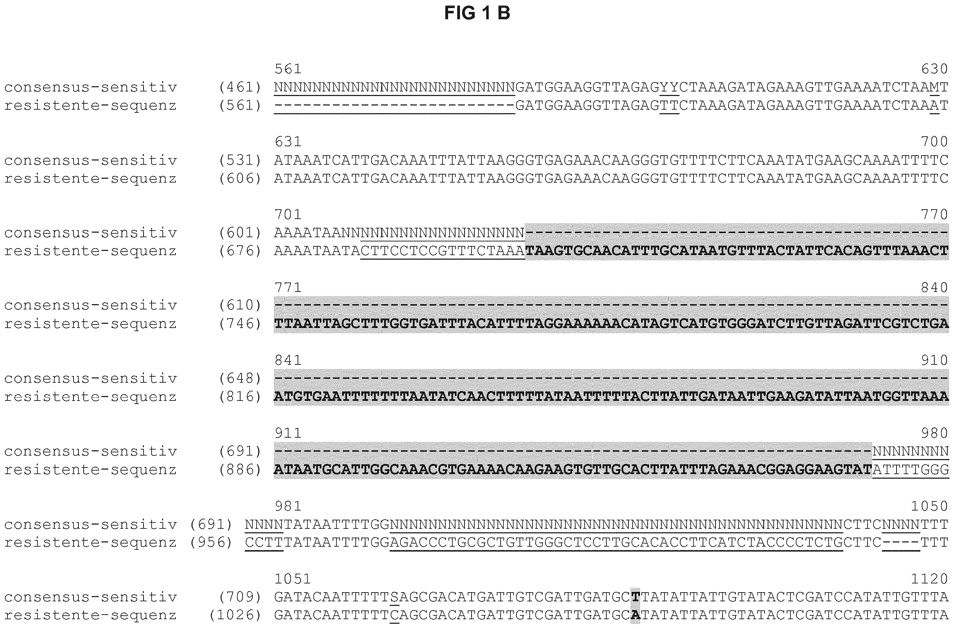

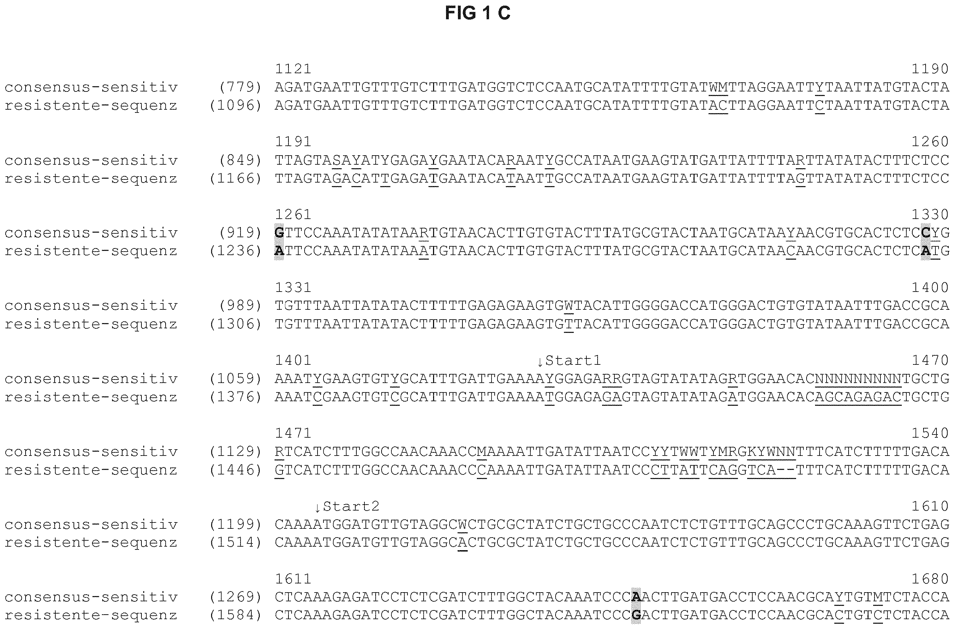

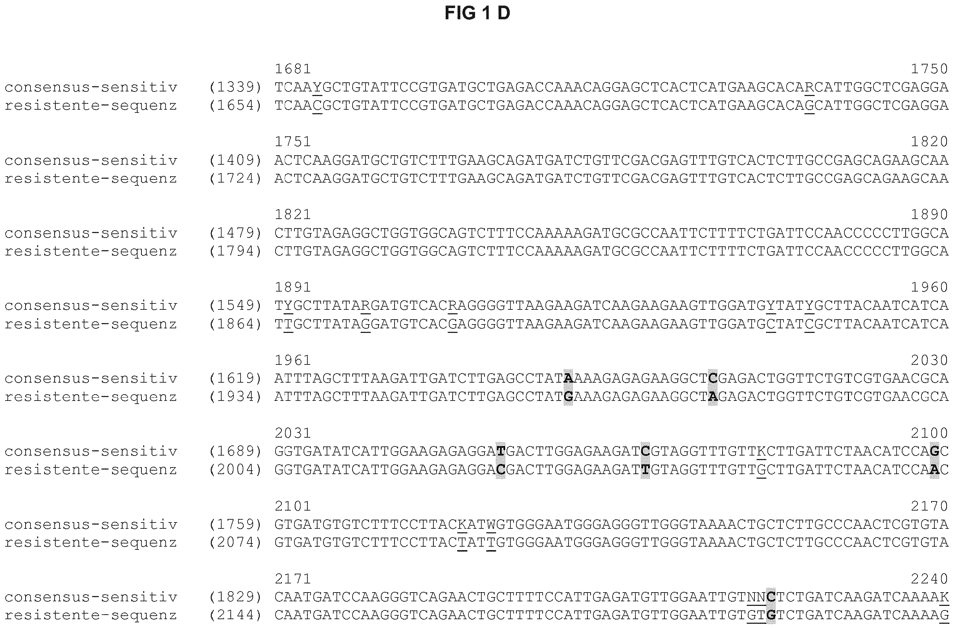

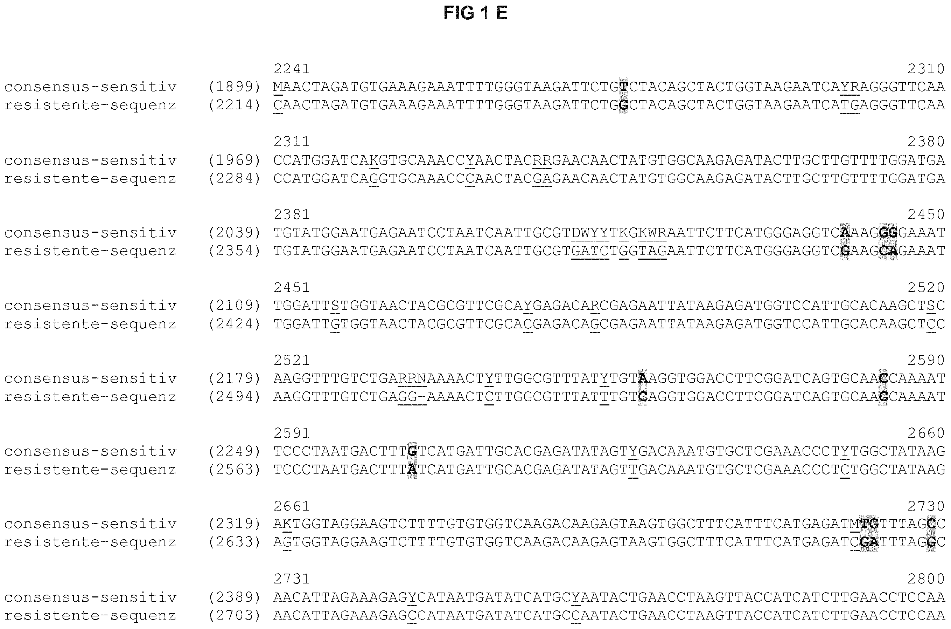

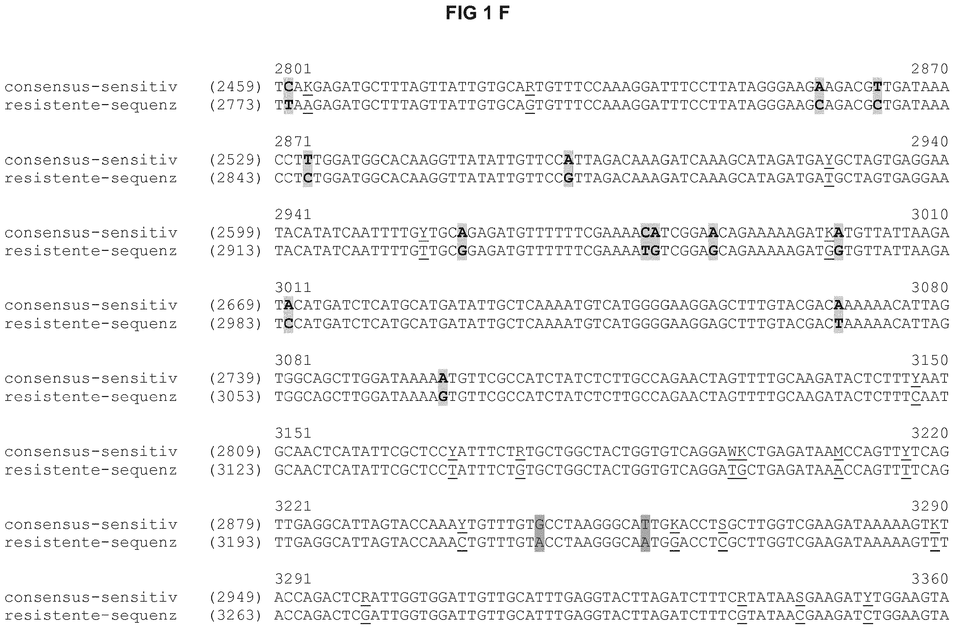

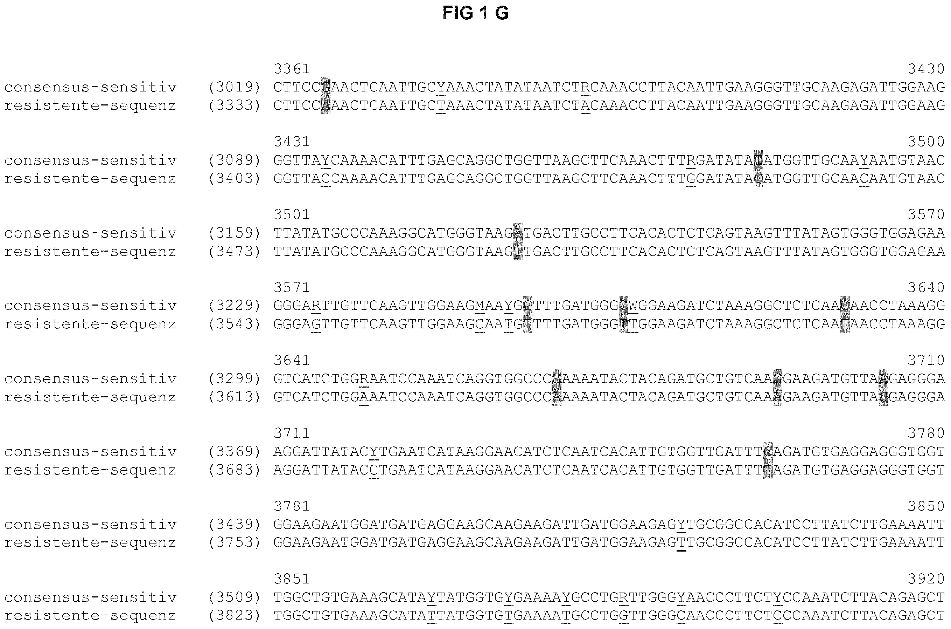

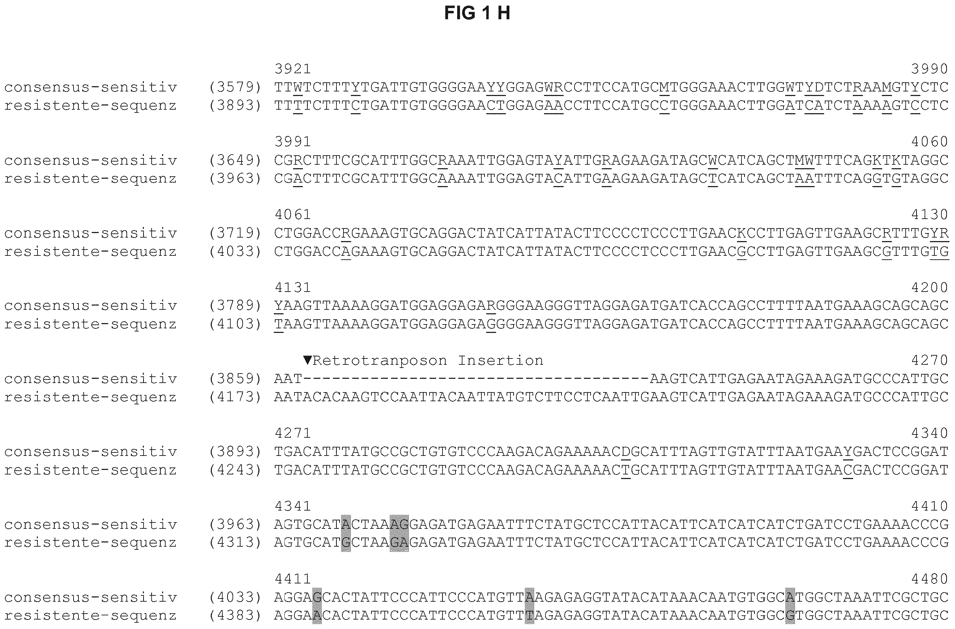

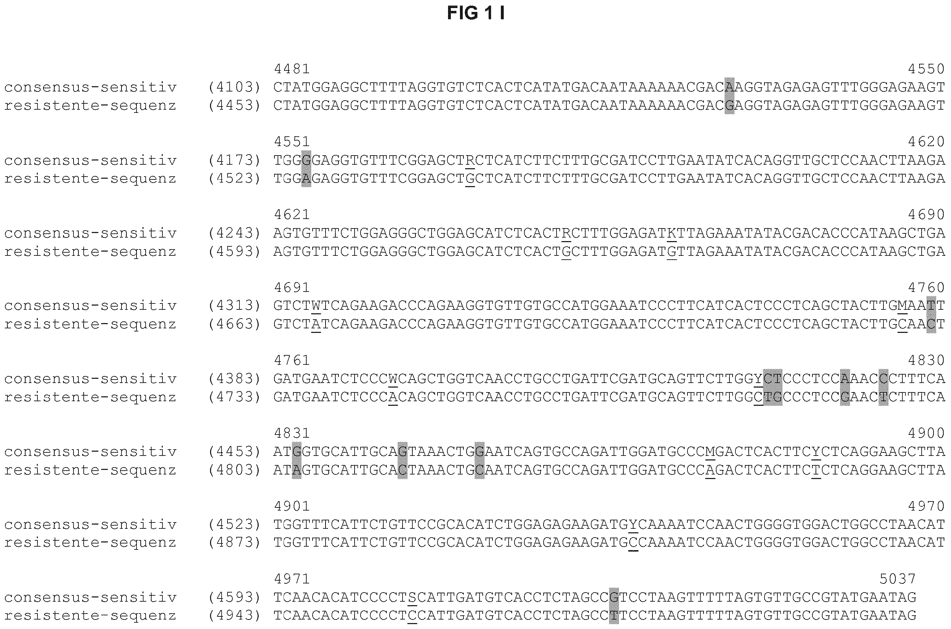

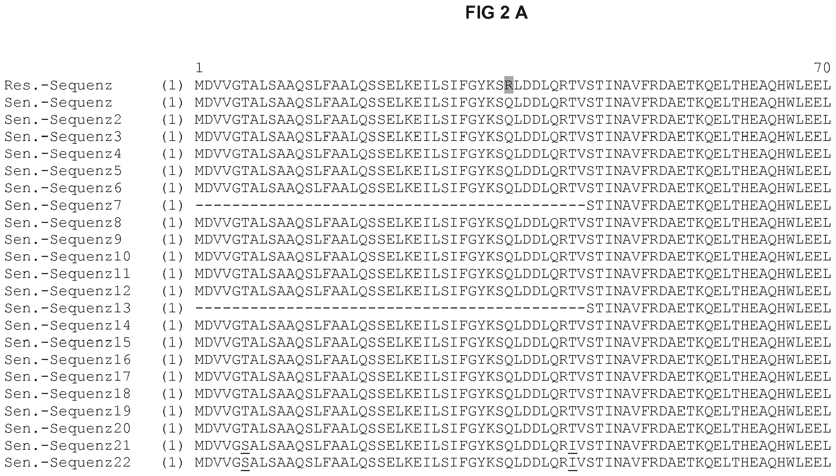

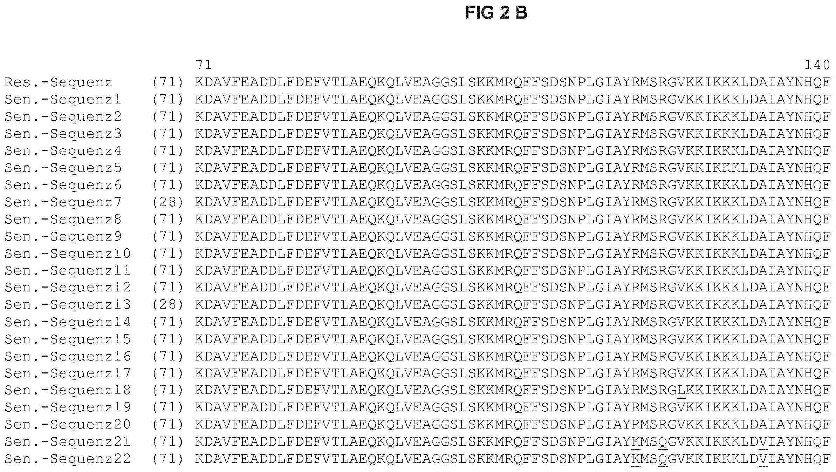

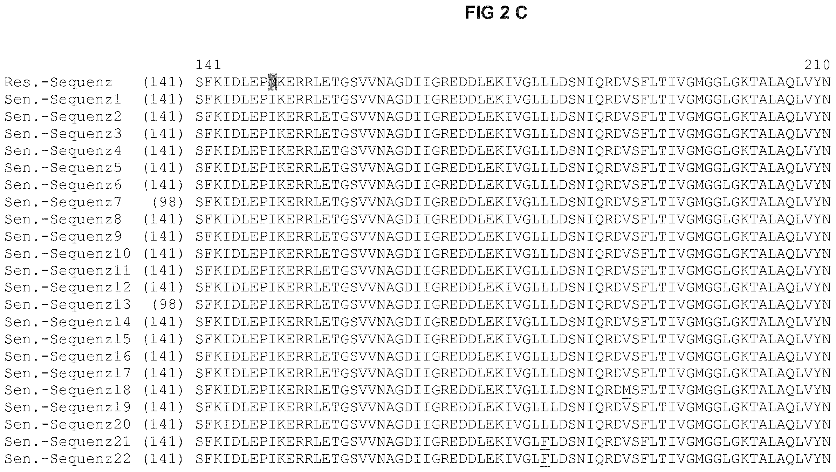

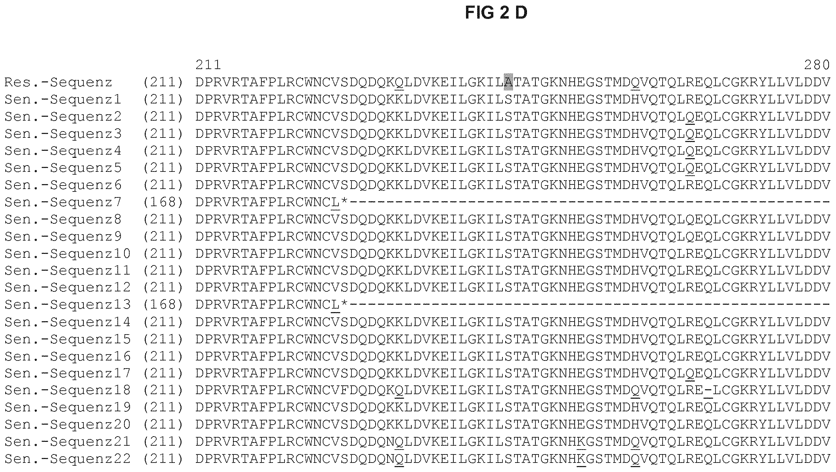

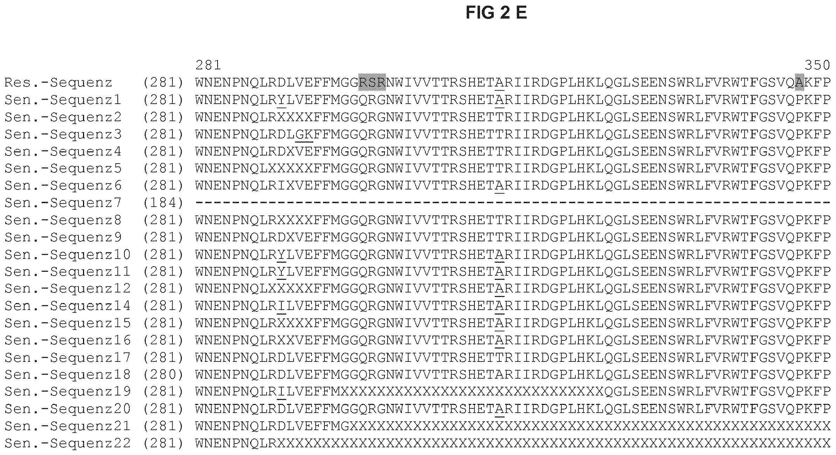

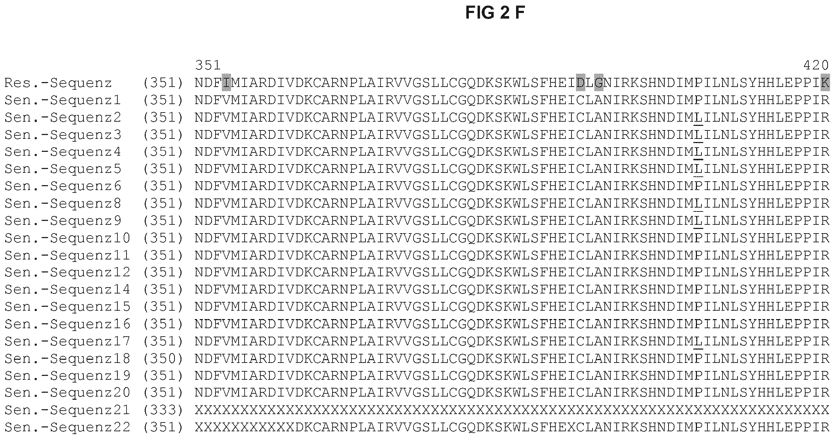

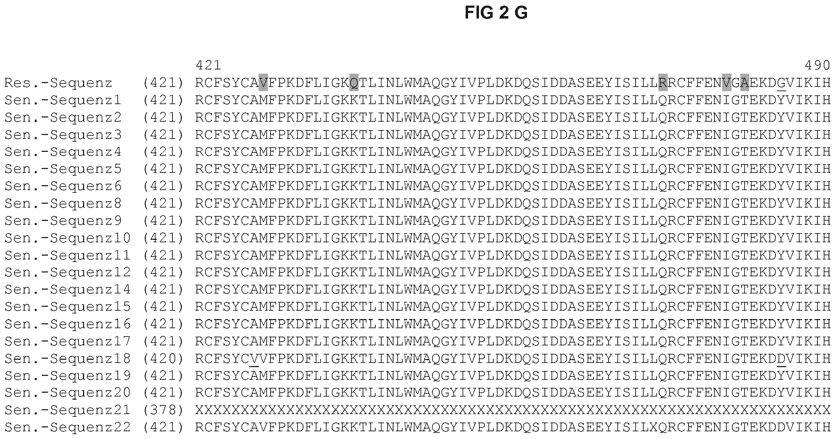

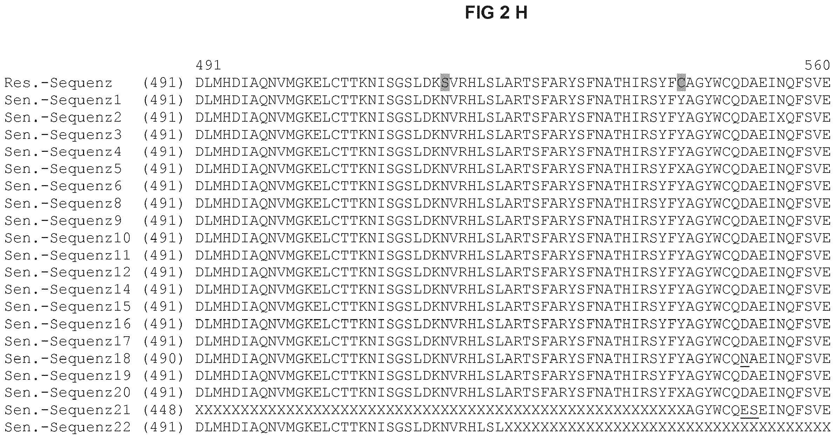

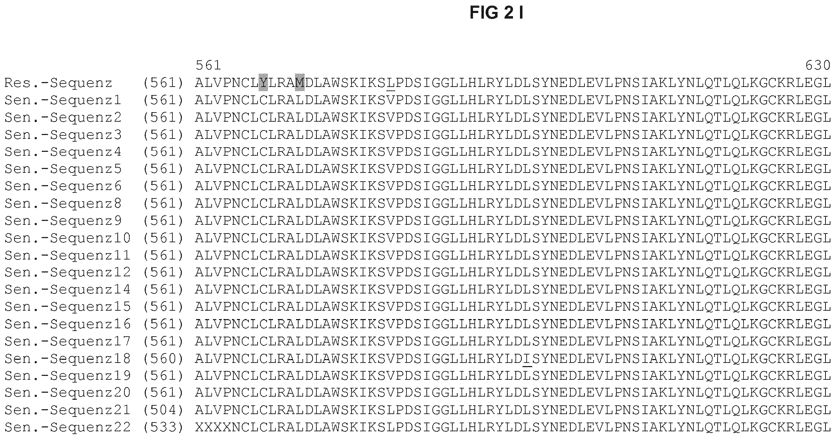

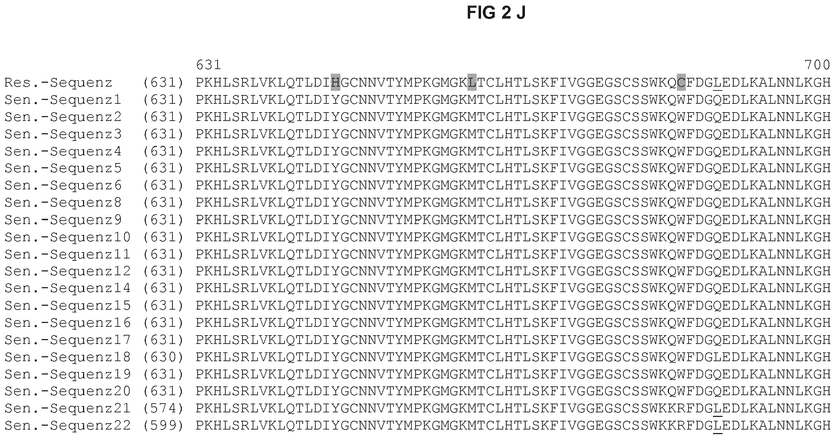

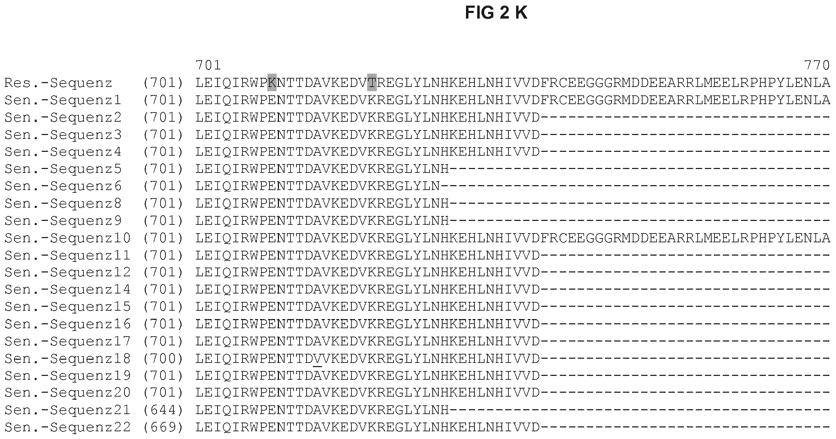

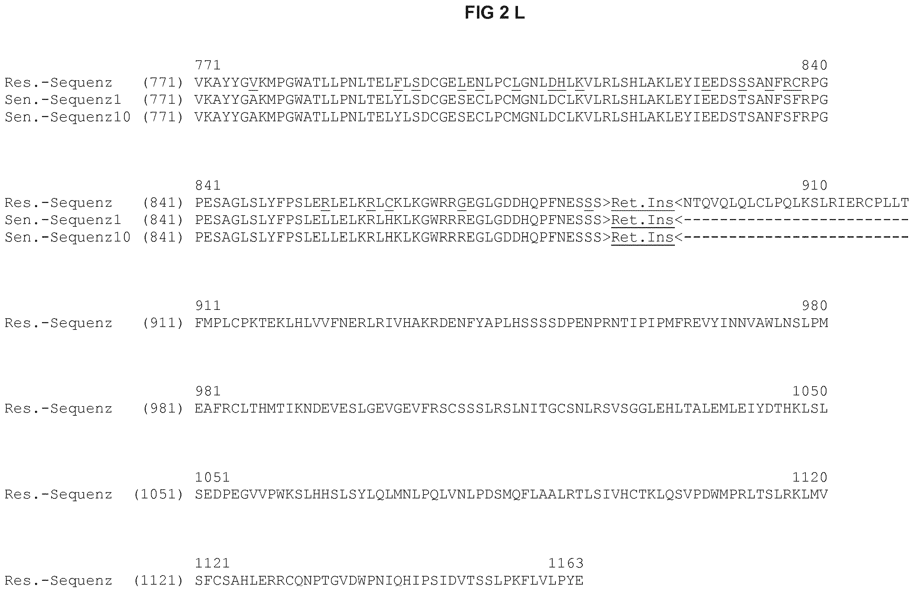

Der Vergleich des NBS-LRR-Gens in ss-Genotypen mit demjenigen in RR-Genotypen zeigte ferner diagnostische Polymorphismen, welche den Figuren 1, 2 und 3 entnommen werden können. Basierend auf diesen Polymorphismen im NBS-LRR Gen wurden Marker entwickelt und in einem breiten Set von nahezu 100 ss-und RR-Genotypen getestet. Die Markermuster aber auch die vergleichende Sequenzierung im Zielgen haben bestätigt, dass die Insertion nahezu immer mit der Sensitivität gekoppelt ist. Es fanden sich jedoch wenige ss-Genotypen, die die Retrotransposon-Insertion nicht aufwiesen und dennoch anfällig waren. Diese ss-Genotypen konnten jedoch eindeutig mittels Marker, welche die diagnostischen Polymorphismen gemäß Figuren 1, 2 und/oder 3 beschreiben, von den RR-Genotypen unterschieden werden.

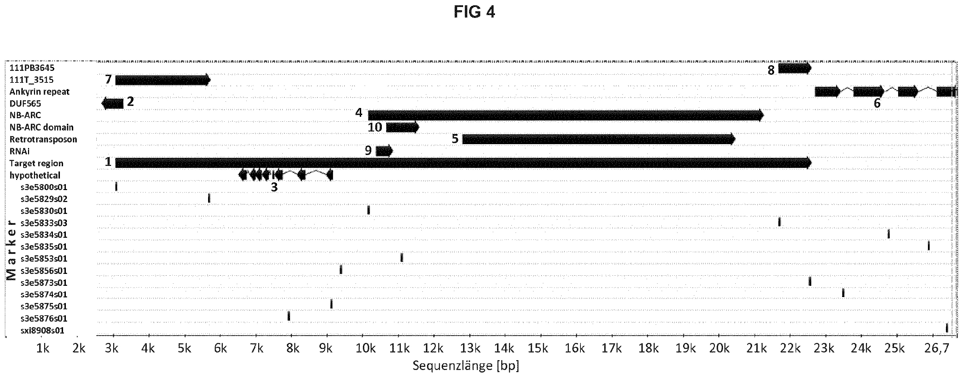

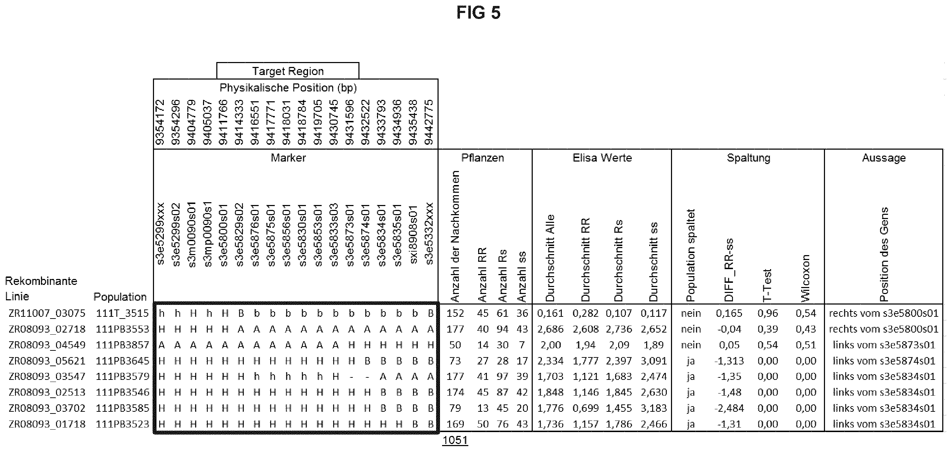

In der analysierten Population wurden Rekombinaten in der Zielregion identifiziert, die eine Rekombination zwischen dem NBS-LRR-Gen und dem stromabwärts gelegenen, benachbarten annotierten putativen Gen, das für ein Ankyrin repeat Protein kodieren könnte, zeigen. Im Falle von zwei Pflanzen sind die Rekombinationen zwischen dem NBS-LRR-Gen und dem stromaufwärts gelegenen, benachbarten annotierten putativen Gen, das für ein DUF565 Protein (Protein mit unbekannter Funktion) kodieren könnte, zu finden. Durch die Resistenzanalyse der Nachkommenschaft von allen diesen rekombinanten Pflanzen (Ein-Gen-Entfernung stromaufwärts und stromabwärts des NBS-LRR-Gens) konnte ganz eindeutig bewiesen werden, dass das Gen zwischen dem Ankyrin repeat Gen und dem DUF565-Gen, nämlich das hier charakterisierte NBS-LRR-Gen für die Resistenz im RR-Genotypen verantwortlich ist. Figur 4 zeigt die physikalische Karte der RZ-3-Zielregion mit den entwickelten Markern. Die Genotypdaten von acht engen rekombinanten Linien, sowie die statistische Analyse deren Nachkommenschaft ist in der Figur 5 dargestellt.

Weiterhin offenbart wird ein Verfahren zur Identifikation eines Nukleinsäuremoleküls, welches ein Protein kodiert, das in der Lage ist, eine Resistenz gegenüber dem Pathogen BNYVV in einer Pflanze der Gattung Beta, in welcher das Protein exprimiert wird, zu vermitteln. Das Verfahren umfasst das Nachweisen der Abwesenheit einer Insertion in der kodierenden Nukleotidsequenz des Nukleinsäuremoleküls. Bevorzugt umfasst das Verfahren das Nachweisen der Abwesenheit einer Insertion, insbesondere eines Retrotransposons, in der kodierenden Nukleotidsequenz des Nukleinsäuremoleküls. Das Retrotransposon kann beispielsweise etwa 500 bp, etwa 1000 bp, etwa 2000 bp, etwa 4000 bp, etwa 8000 bp oder mehr als etwa 8000 bp lang sein. In einer besonderen Ausgestaltung des Verfahrens ist das Nukleinsäuremolekül das Nukleinsäuremolekül wie oben beschrieben und kodiert das Resistenz-vermittelnde RZ-3-Gen oder ein funktionelles Homolog von RZ-3. Vorzugsweise handelt es sich bei der Pflanze der Gattung Beta um *Beta vulgaris* subsp. *maritima* oder *Beta vulgaris* subsp. *vulgaris* var. *altissima* (Zuckerrübe). Dem Fachmann ist bekannt, welche Methoden geeignet sind, die Abwesenheit der Insertion nachzuweisen. Beispielsweise kann der Fachmann in Kenntnis der hier offenbarten Nukleinsäuremoleküle molekulare Marker entwickeln, welche die Anwesenheit oder die Abwesenheit einer Insertion in oben beschriebener Region im NBS-LRR-Gen nachweisen (exemplarisches Vorgehen siehe Beispiele). Die vorliegende Offenbarung schließt solche Marker sowie deren Verwendung zum Nachweis der An- oder Abwesenheit der Insertion zur Selektion resistenter, inbesondere BNYVV-resistenter, Pflanzen, insbesondere *Beta vulgaris* subsp. *maritima* oder *Beta vulgaris* subsp. *vulgaris* var. *altissima* (Zuckerrübe) mit ein. Vorzugsweise beschreiben solche Marker Loki an den Insertionsstellen des Retrotransposons. Insertionsstellen meinen Übergangstellen zwischen genomischer DNA und Retrotransposon auf 5`- und/oder 3'-Seite der Insertion. Übergangsstellen sind breit zu definieren und Markerloki können weniger als 1000 Nukleotide, bevorzugt weniger als 800 oder 600 Nukleotide, besonders bevorzugt weniger als 400, 200, 150, 100, 50, 40, 30, 20 oder 10 Nukleotide stromaufwärts oder stromabwärts von einer Insertionsstelle auf der DNA entfernt liegen. Alternativ oder ergänzend zu dem Schritt des Nachweises der An- oder Abwesenheit einer Insertion in der kodierenden Nukleotidsequenz des Nukleinsäuremoleküls, kann das Verfahren auch das Nachweisen von mindestens einem Polymorphismus gemäß Figur 1, 2 und/oder 3, bevorzugt von mindestens zwei oder drei Polymorphismen gemäß gemäß Figur 1, 2 und/oder 3, besonders bevorzugt von mindestens vier, fünf oder mehr Polymorphismen gemäß gemäß Figur 1, 2 und/oder 3 in der kodierenden Nukleotidsequenz des Nukleinsäuremoleküls unter Verwendung von molekularen Markern, welche die Polymorphismen, insbesondere diagnostische Polymorphismen, erkennen, umfassen. Bevorzugt findet dieser Nachweis unter Verwendung von mindestens einem molekularen Marker pro Polymorphismus, insbesondere pro diagnosistischen Polymorphismus, statt. Dem Fachmann ist bekannt, welche Markertechniken zum Nachweis eines entsprechenden Polymorphismus anzuwenden sind, und wie man molekulare Marker dafür konstruiert (Literatur). Weiterhin offenbart sind molekulare Marker, welche einen Polymorphismus gemäß Figur 1, 2 und/oder 3 beschreiben bzw. detektieren, wie auch die Verwendung eines molekularen Markers zur Detektion eines Polymorphismus gemäß Figur 1, 2 und/oder 3. Weiterhin stellen die vorstehenden Identifikationsverfahren auch Verfahren zur Selektion einer Pflanze, welche eine Resistenz gegenüber BNYVV aufweist, dar. Das Verfahren zur Selektion umfasst einen abschließenden Schritt des Selektierens einer resistenten Pflanze.

Ferner konnte auch gezeigt werden, dass in der untersuchte RR Genotypen stromaufwärts zu RZ-3 (SEQ ID NO: 1) angrenzende genomische DNA-Sequenzabschnitt gemäß SEQ ID NO: 4 und stromabwärts zu RZ-3 (SEQ ID NO: 1) angrenzende genomische DNA-Sequenzabschnitt gemäß SEQ ID NO: 5 aufwies, welche eng-gekoppelt mit dem RZ-3-Gen sind und sich deshalb herausragend eignen als DNA-Regionen zur Entwicklung von diagnostischen Markern für RZ-3. Daher umfasst die vorliegende Offenbarung ein Verfahren zur Selektion einer Pflanze, welche eine Resistenz gegenüber BNYVV aufweist. Das Verfahren zur Selektion umfasst die Verwendung eines molekularen Markers auf einer DNA-Sequenz gemäß SEQ ID NO: 4 und/oder auf einer DNA-Sequenz gemäß SEQ ID NO: 5 und einen abschließenden Schritt des Selektierens einer resistenten Pflanze. Dem Fachmann ist bekannt, wie er auf Grundlage der offenbarten Sequenzinformationen Marker entwickelt und einsetzt.

Durch die vorliegende Erfindung können ferner folgende Vorteile für die Züchtung und die Entwicklung neuer resistenter Pflanzenlinien der Gattung Beta erzielt werden: Sequenzinformationen sowie die identifizierten Polymorphismen, welche eine Unterscheidung zwischen resistenten RR- und anfälligen ss-Allelen des offenbarten Gens erlauben, machen die Marker-Entwicklung direkt im Gen möglich, was insbesondere im Hinblick auf die Entwicklung von optimierten Elitelinien ohne "Linkage Drag" eine bedeutende Erleichterung für den Pflanzenzüchter darstellt. Außerdem kann die Kenntnis über die sequentielle Struktur zur Identifizierung von weiteren Resistenzgenen, insbesondere gegen Rizomania, welche beispielsweise teilweise homolog sind, verwendet werden.

Die hier offenbarte Nutzung des resistenten Genallels in cis- oder trans-genetische Ansätzen eröffnet die Möglichkeit neue resistente Sorten der Gattung Beta zu entwickeln, welche eine anhand des Dosiseffekts eine höhere Resistenz aufweisen oder in welchen durch das Stacking des offenbarten Gens mit anderen Resistenzgenen ein Resistenzbruch vermieden werden und die Resistenzausprägung optimiert werden kann. Ferner sind Modifikationen des Gens mittels Tilling oder gezieltem Engineering zur Entwicklung neuer Resistenzallele denkbar.

Ferner offenbart ist die Verwendung des identifizierten resistenten RZ3-Genallels in einem genetischen oder molekularen Stack mit anderen genetischen Elementen, welche agronomisch vorteilhafte Eigenschaften vermitteln können, in einer Pflanze. Hierdurch kann der ökonomische Wert von Kulturpflanzen deutlich gesteigert werden, indem beispielsweise die Ertragsleistung gesteigert wird oder neue Anbauflächen für eine Pflanze erschlossen werden, die zuvor unter Anderem aufgrund biotischen Faktoren wie starkem Pathogendruck oder abiotischen Faktoren wie Trockenheit dem Anbau dieser Pflanze nicht zugänglich waren. Eine agronomisch vorteilhafte Eigenschaft ist beispielsweise eine Toleranz gegenüber einem Herbizid wie Glyphosat, Glufosinat oder ALS-Inhibitoren. Dem Fachmann sind aus dem Stand der Technik zahlreiche weitere Herbizide und deren Anwendbarkeit bekannt. Er kann auf den Stand der Technik zurückgreifen, um Kenntnis zu erlangen, welche genetischen Elemente in welcher Art und Weise zu verwenden sind, um eine entsprechende Toleranz in Pflanzen zu implementieren. Ein weiteres Beispiel für eine agronomisch vorteilhafte Eigenschaft ist eine zusätzliche Pathogenresistenz, wobei Pathogene beispielsweise Insekten, Viren, Nematoden, Bakterien oder Fungi sein können. Durch Kombination unterschiedlicher Pathogenresistenzen/-toleranzen kann beispielsweise eine breite Pathogenabwehr für eine Pflanze erreicht werden, da genetische Elemente untereinander ergänzende Wirkungen aufweisen können. Hierfür sind dem Fachmann als genetische Elemente beispielsweise zahlreiche Resistenzgene bekannt. Ein weiteres Beispiel für eine agronomisch vorteilhafte Eigenschaft ist eine Kühle- oder Frosttoleranz. Pflanzen, welche diese Eigenschaft aufweisen, könnten früher im Jahr bereits ausgesät werden oder könnten länger zum Beispiel auch über Frostperioden im Feld verbleiben, was beispielsweise zu gesteigerten Erträgen führen kann. Auch hier kann der Fachmann auf den Stand der Technik zurückgreifen, um geeignete genetische Elemente zu finden. Weitere Beispiele für agronomisch vorteilhafte Eigenschaften sind Wassernutzungseffizienz, Stickstoffnutzungseffizienz sowie Ertrag. Genetische Elemente, welche eingesetzt werden können, um solche Eigenschaften zu vermitteln, könnten im Stand der Technik gefunden werden.

Dem Fachmann sind weiterhin zahlreichen Modifikationen zur Pathogenabwehr bekannt. Neben den häufig beschriebenen Familien der R-Gene könnten der Avr/R-Ansatz, die Avr-Gen-Komplementierung (WO 2013/127379), die Autoaktivierung eines R-Gens (WO 2006/128444), der HIGS (host induced gene silencing)-Ansatz (z.B. WO2013/050024) oder der VIGS (virus induced gene silencing)-Ansatz vorteilhaft eingesetzt werden. Insbesondere die Autoaktivierung eines R-Gens könnte für die vorliegende Erfindung von Bedeutung sein. Hierfür ist eine Nukleinsäure zu erstellen, die für ein autoaktiviertes Resistenzprotein zur Erzeugung einer Resistenz gegenüber Pathogenen bei Pflanzen kodiert. Diese Nukleinsäure weist dann nur einen begrenzten Teil eines NBS-LRR-Resistenzgens wie das RZ3-Gen aufweist, der sich vom 5'-Ende des kodierenden Bereichs des NBS-LRR-Resistenzgens stromabwärts bis zum Beginn der NBS-Domäne des NBS-LRR-Resistenzgens erstreckt, wobei das NBS-LRR-Resistenzgen kein TIR-NBS-LRR-Resistenzgen ist.

Ferner schließt die Offenbarung auch die Verwendung des resistenten RZ3-Genallels, identifiziert mit einem oben beschriebenen Verfahren, zur Kombination mit einer vorstehenden Modifikationen oder mit einem vorstehend beschriebenen genetischen Element, welches eine oder mehrere agronomisch vorteilhafte Eigenschaften in einer Pflanze vermitteln kann.

Ausgestaltungen und Ausführungsformen der vorliegenden Erfindung werden in exemplarischer Weise mit Bezug auf die angehängten Figuren und Sequenzen beschrieben:

### Sequenzen:

| | |
|---|---|
| SEQ ID NO: 1 | genomische DNA-Sequenz des Resistenzgens RZ-3. Die Sequenz umfasst von Nukleotid 1 bis 1403 die regulatorische Region des Promotors |
| SEQ ID NO: 2 | vorhergesagte Proteinsequenz des Resistenzproteins RZ-3_1 |
| SEQ ID NO: 3 | vorhergesagte Proteinsequenz des Resistenzproteins RZ-3_2 |

| | |
|---|---|
| SEQ ID NO: 4 | stromaufwärts zu RZ-3 (SEQ ID NO: 1) angrenzende chromosomale Region |
| SEQ ID NO: 5 | stromabwärts zu RZ-3 (SEQ ID NO: 1) angrenzende chromosomale Region |
| SEQ ID NO: 6 | Consensus-Sequenz der genomischen Sequenz des RZ-3-Gens in ss Genotypen |
| SEQ ID NO: 7 | Zielsequenz im RZ3-Gen des RNAi-Konstrukts im Vektor pZFN-C48-RNAi. |

### Figuren:

Fig. 1 A-I: Nukleotidsequenzvergleich zwischen Consensus-Sequenz der genomischen Sequenz des RZ-3-Gens in ss Genotypen (SEQ ID NO: 6) und dem RZ-3-Gen der RR-Genotypen (SEQ ID NO: 1). Diagnostische Polymorphismen sind grau hinterlegt und fett gedruckt hervorgehoben. Unterstrichen sind solche Polymorphismen, welche nicht diagnostisch sind. Die potentiellen Transkriptionsstartpunkte in dem gen sind mit Pfeilen gekennzeichnet. Sie führen zu zwei Polypeptid-Varianten RZ-3_1 und RZ-3_2. Die Stelle des Retrotransposons ist mit einem schwarzen Dreieck auf der Spitze gekennzeichnet.
Fig. 2 A-L: Aminosäuresequenzvergleich des vorhergesagten Polypeptids aus den RR-Genotypen (RZ-3_1; SEQ ID NO: 2) und Polypeptiden aus 22 unterschiedlichen ss-Genotypen. Diagnostische Polymorphismen sind grau hinterlegt und fett gedruckt hervorgehoben. Unterstrichen sind solche Polymorphismen, welche nicht diagnostisch sind.

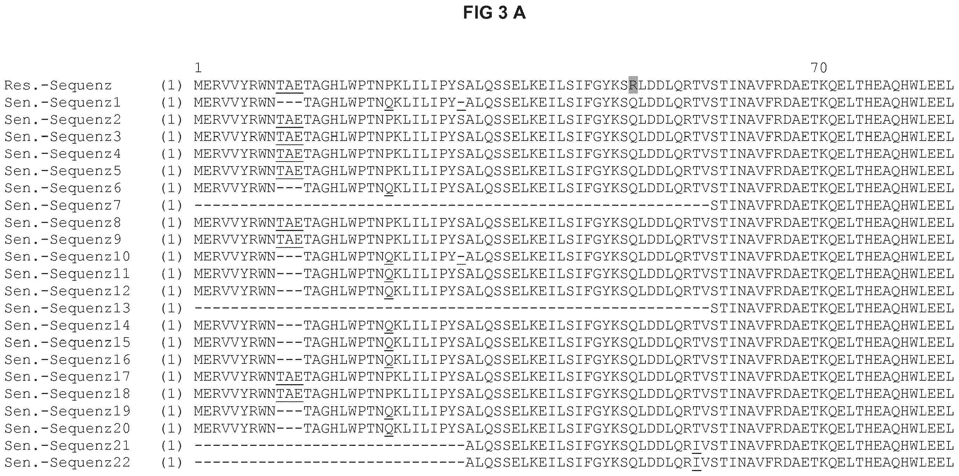

Fig. 3 A-L: Aminosäuresequenzvergleich des vorhergesagten Polypeptids aus den RR-Genotypen (RZ-3_2; SEQ ID NO: 3) und Polypeptiden aus 22 unterschiedlichen ss-Genotypen. Diagnostische Polymorphismen sind grau hinterlegt und fett gedruckt hervorgehoben. Unterstrichen sind solche Polymorphismen, welche nicht diagnostisch sind.
Fig. 4: Physikalische Karte der RZ-3-Zielregion. In der dargestellten Zielregion des sensitiven Referenzgenotyps wurden fünf Gene annotiert ("2" (DUF565), "3" (hypothetisches Protein), "4" (NBS-LRR Kandidatengen), "5" (Retrotransposon) und "6" (Ankyrin-Repeat)). Das NBS-LRR Kandidatengen ("4") beinhaltet in der sensitiven Referenz-Sequenz eine Retrotransposon-Insertion ("5"). Dieses Retrotransposon fehlt vollständig in der resistenten Sequenz, so dass in dem resistenten Genotyp nur noch vier Gene annotiert werden können ("2", "3", "4" und "6"). Die Positionen der engsten Rekombinationen (Rekombinanten: 111T_3515/ ZR11007_03075 mit Ziffer "7" und 111 PB3645/ZR08093_05621 mit Ziffer "8") sind oberhalb dargestellt. Mit deren Hilfe konnte die kürzere Zielregion "1" eingrenzt werden. Die hierfür entwickelten Marker aus der Rekombinanten-Analyse sind als schwarze Striche im unteren Teil der Figur wiedergegeben. Zur Validierung des Gens im RNAi-Ansatz wurde für das *Gene silencing* des resistenten RZ-3-Genallels ein Gensegment ("9") teilweise aus dem Domainbereich "10" als Zielsequenz ausgewählt.
Fig. 5: Markeranalyse der engsten Rekombinanten in der RZ-3 Zielregion (kleine Buchstaben im fett-gerahmten Bereich sind *in silico* generierte Markerdaten). Die acht rekombinanten Linien wurden mit insgesamt 1051 Nachkommen phänotypisiert und genotypisiert. Die Nachkommen wurden anhand der Markerdaten im NBS-LRR Kandidatengen bzw. der spaltenden flankierenden Region im Falle, dass das NBS-LRR Kandidatengen homozygot RR bzw. ss ist, in 3 Gruppen eingeteilt (RR-resistent homozygot, Rs - hetrozygot, ss-sensitiv homozygot). Daneben sind die entsprechenden ELISA-Werte wiedergegeben. Eine Spaltung oder Nicht-Spaltung der Nachkommen wurde mit T-test und Wilcoxon Statistik nach Signifikanz überprüft. Anhand der Ergebnisse konnte das Kandidatengen ganz eindeutig zwischen den Markern s3e5800s01 und s3e5873s01 eingegrenzt werden.

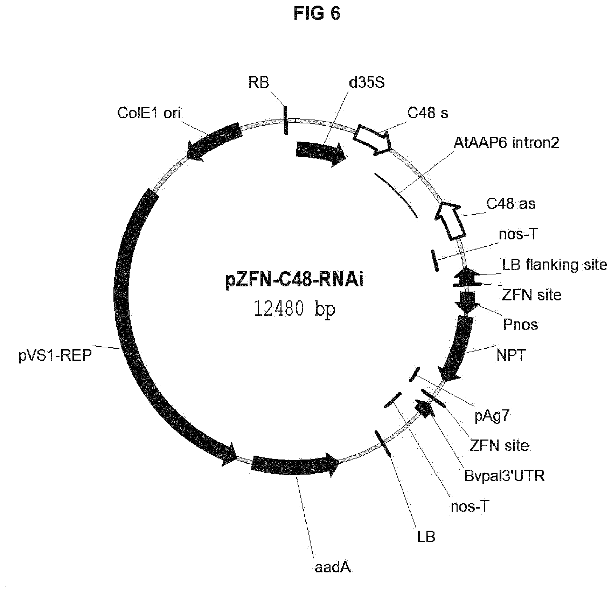

Fig. 6: Transformationsvektor pZFN-C48-RNAi: d35S-promoter; C48 s: C48 sequence sense orientation; AtAAP6 intron2: Arabidopsis thaliana amino acid permease 6 intron; C48 as: C48 sequence antisense orientation; Nos-T: nos Terminator; LB flanking site: Left border flanking site; ZFN site: Zinc-finger nuclease recognition site (complementary); Pnos: Nos promoter; NPT: coding sequence; neomycin phosphotransferase (npt) gene; pAG7: pAG7 terminator; Bvpal3'UTR: 3' untranslated region of the Beta vulgaris Pal gene; LB: Left border; aadA: coding sequence; aminoglycoside-3"-adenylyltransferases (AAD); pVS1-REP: pVS1 replication origin; ColE1 ori: ColE1 replication origin; RB: right border.

### Examples:

### Kartierung und Feinkartierung des RZ-3-Gens / Genetische-Physikalische Karte

Die RZ-3-Resistenz (auch als C48-Resistenz oder C48 bezeichnet) wurde in mehreren Schritten mittels Kartierung und Feinkartierung auf dem Chromosom 3 zwischen 57,1 und 57,8 cM (interne Referenzkarte) kartiert, also auf einer genetische Distanz zwischen zwei flankierenden Markern von 0,0714 cM in der genetischen Karte. Für die Kartierung wurden insgesamt 8004 Pflanzen aus der Kreuzung S504 (sensitiver Genotyp) x T74 (resistenter Genotyp) untersucht. Parallel zu der C48 QTL Kartierung wurden nach jedem Kartierungsschritt zielorientiert neue informative Marker entwickelt und für die Einschränkung der C48 Zielregion eingesetzt.

Die Feinkartierungskoordinaten wurden zusätzlich mit der Analyse der Nachkommen der informativen Rekombinanten bestätigt. Informative rekombinante BC2S1 oder BC2S2 Pflanzen wurden dazu intensiv mit jeweils 90-180 Nachkommen analysiert. Diese Nachkommen wurden einzelpflanzenweise genotypisiert und parallel phänotypisiert. Mittels statistischer Verfahren (t-Test, Poweranalyse) wurden die Phänotypen der informativen Rekombinanten (homozygot resistent-RR / heterozygot-Rs / homozygot anfällig-ss) nachgewiesen und damit Rückschlüsse auf den Genotyp der informativen Rekombinanten gezogen. Sofern sich die Homozygotenklassen der Nachkommen (RR versus ss) in der Resistenz unterscheiden, liegt das Gen im heterozygoten Bereich (Rs) der Elternpflanze; andernfalls im homozygoten Bereich (RR oder ss) der Elternpflanze.

Eine physikalische Karte wurde generiert für einen Rizomania-sensitiven Genotyp, indem Marker und deren genetische Positionen auf die Chromosomsequenzen projiziert worden sind. Mit der Einschränkung der C48 QTL-Region wurden anhand der Referenz-Sequenz und zusätzlichen vergleichenden Sequenzierungen in resistenten Genotypen (Next Generation Sequenzierung und Sanger Sequenzierung) neue informative Marker entwickelt.

Die durch die Feinkartierung identifizierte Region umfasst eine Sequenzlänge von 37996 Basenpaaren (Positionen flankierender SNP-Marker) in der sensitiven Referenz-Sequenz. Die Kollinearität zwischen den genetischen und der Physikalischen Karte in dem Zielbereich ist widerspruchsfrei (Reihenfolge von 12 Markern im Zielbereich).

### Identifizierung und Sequenzierung von resistenten BAC Klonen

Eine BAC Bibliothek ist entwickelt worden für einen ausgewählten RZ-3 (C48) resistenten Genotyp. Diese BAC Bank wurde mit den angewandten Markern in C48 QTL Bereich durchgemustert. Mehrere BAC Klone wurden für die oben identifizierte Zielregion gefunden. Davon wurden drei unterschiedlich lange BAC-Klone für Sequenzierung ausgewählt, welche vollständig die Zielregion erfassten. Die BAC Klone wurden sequenziert und anhand der entstandenen sequence-reads wurde eine "de novo" Assemblierung durchgeführt. Unter den entstandenen resistenten Sequenzcontigs hatte die grösste Sequenz eine Länge von 110909 bp (34537 reads) und umfasste komplett die Zielregion.

### Vergleich der sensitiven und resistenten Sequenzen - Sequenzauswertung

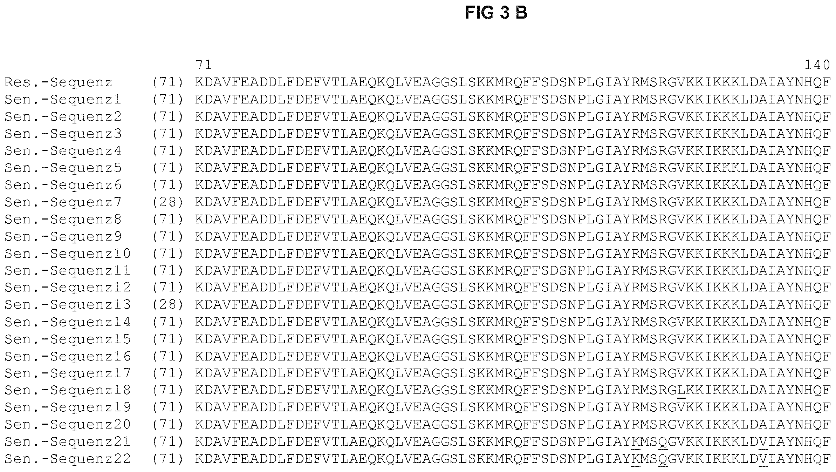

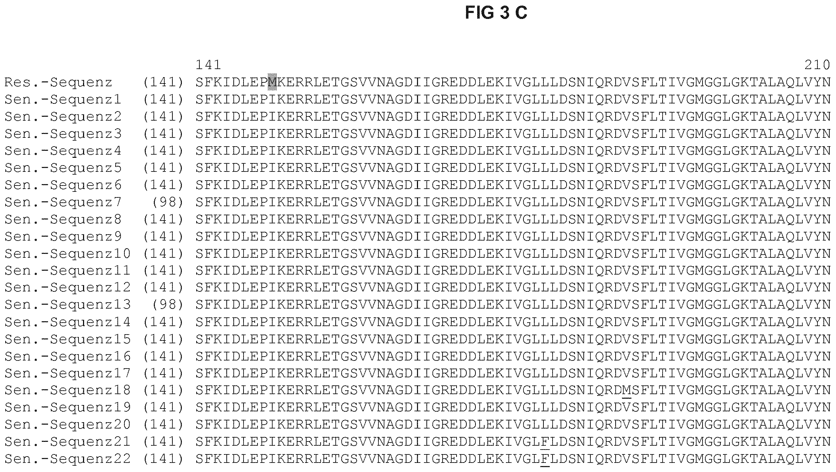

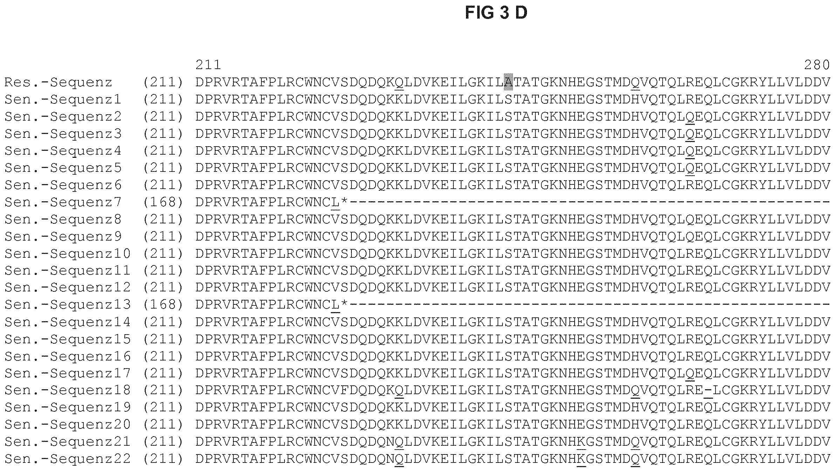

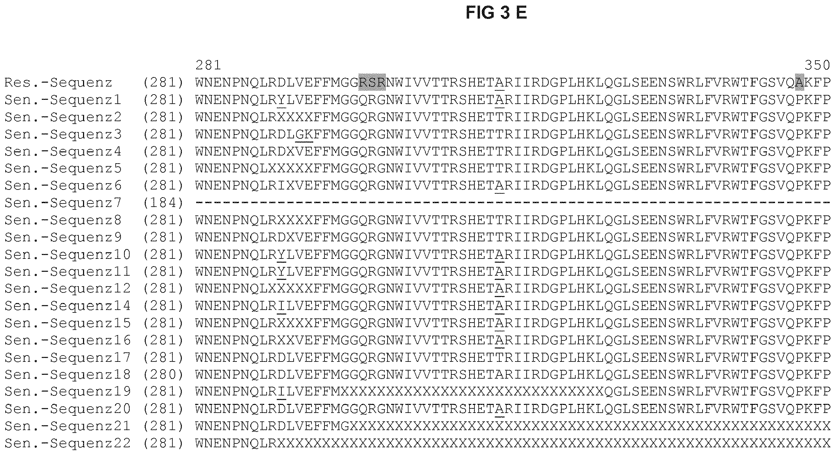

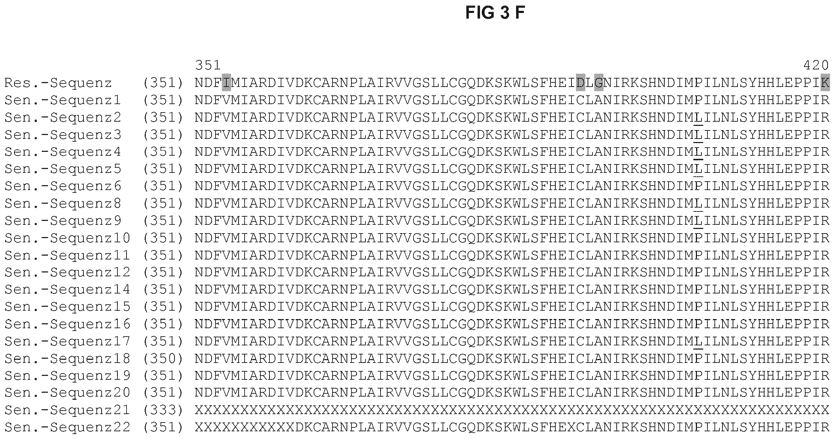

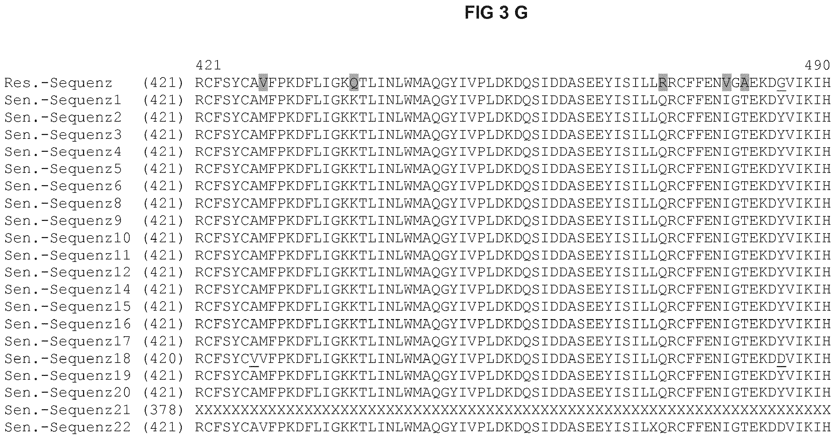

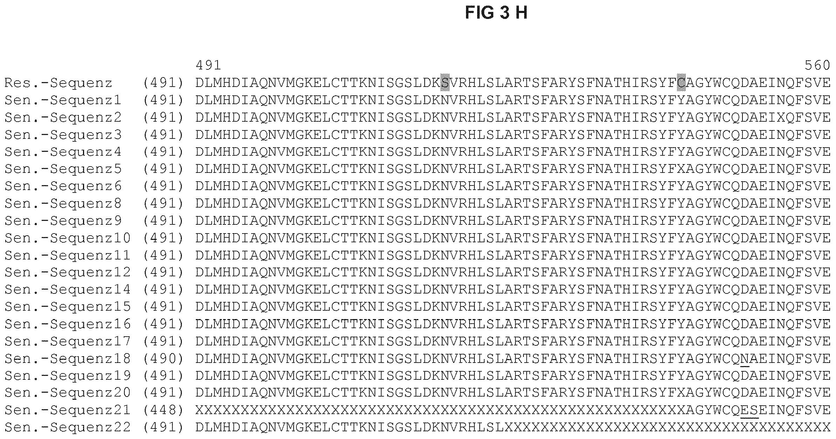

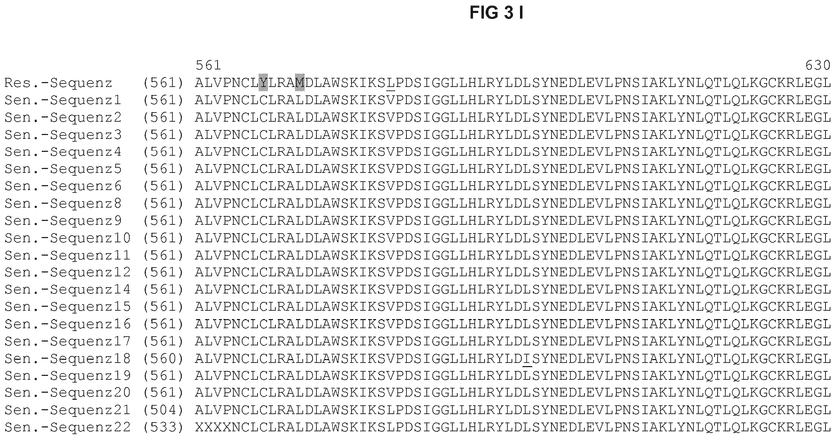

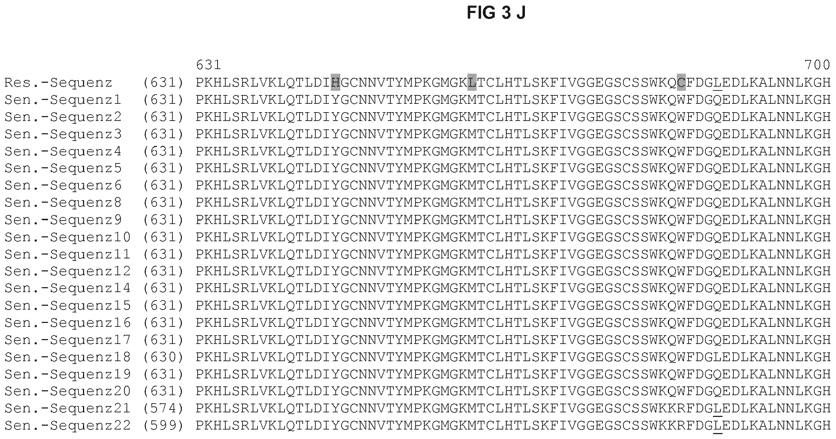

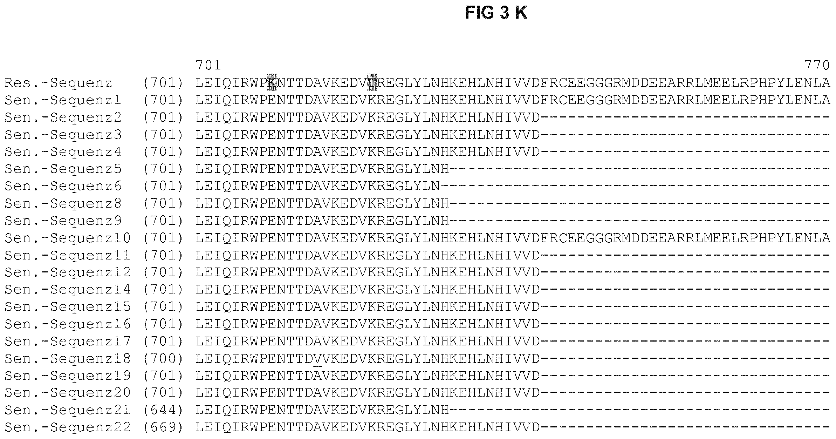

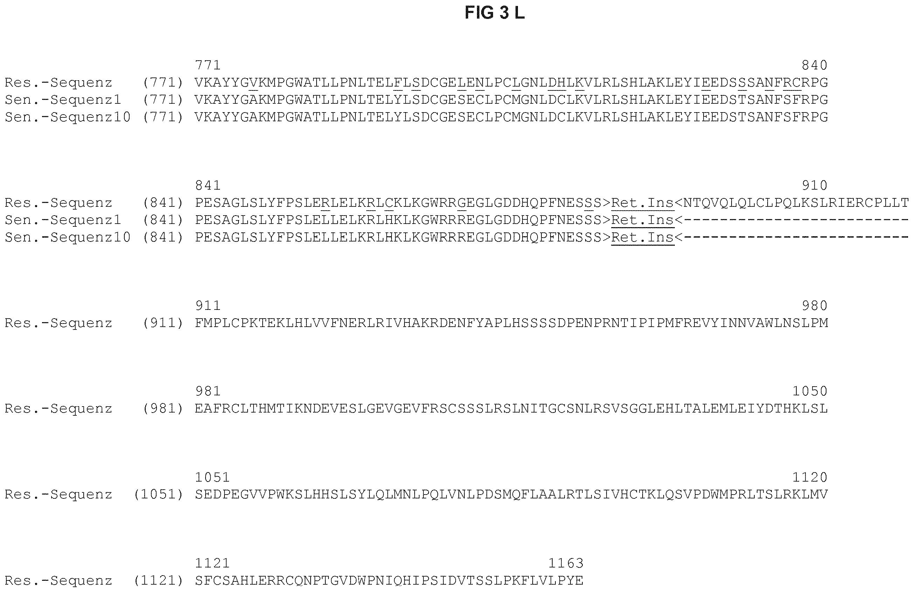

Die Kollinearität der beiden ss- und RR-Sequenzen wurde mit der Anwendung von unterschiedlichen Software-tools verglichen. Für die beiden resistenten und sensitiven Sequenzen wurde eine Genannotation mit den Softwares Maker und Pedant durchgeführt. Die Genannotation auf beiden Sequenzen zeigte die gleiche Abfolge von putativen Genen. Überraschenderweise konnte jedoch ein markanter Unterschied in einem dieser Gene festgestellt werden, nämlich in dem Gen der vorliegenden Erfindung (RZ-3). In dem sensitiven Genotyp konnte in diesem identifizierten NBS-LRR-Gen ein Retrotransposon annotiert werden. Die Insertion des Retrotransposon passierte im Gen zwischen den beiden Domänen der LRR-Domäne und der IR-Domäne. Der resistente Genotyp weist diese Insertion nicht auf und ist in SEQ ID NO: 1 wiedergegeben. Weiterhin wurden anschließend die vorhergesagten Polypeptidsequenzen verglichen und ausgewertet (teilweise dargstellt in siehe Fig. 2 und 3).

### Vergleichende Sequenzierung des NB-ARC-LRR-Kandidatengens

Das NB-ARC-LRR-Kandidatengen wurde in zwei Schritten vergleichend sequenziert. Die Retrotransposon-Insertionsstelle wurde in einem Genotypset mit insgesamt 92 resistenten und sensitiven Genotypen verifiziert. Diese Analyse zeigte, dass alle resistenten Genotypen keine Retrotransposoninsertion hatten. Von den sensitiven Genotypen konnte die Insertion in mehr als 90% der Fälle nachgewiesen werden. Damit scheint der Nachweis der Insertion gekoppelt zu sein mit dem anfälligen Genotyp. Wegen der gefundenen Wiedersprüche (etwa 10% der verbleibenden anfälligen Genotypen ohne Insertion) wurde die Sequenzierung jedoch in dem zweiten Schritt für das gesamte Gen vor der Insertionsstelle mit Promotorbereich ausgeweitet (SEQ ID NO: 1). Insgesamt wurden 31 ausgewählten resistente und anfällige Genotypen inklusive der widersprüchlichen Genotypen sequenziert und verglichen. Im Ergebnis waren alle resistenten Genotypen, die auf sieben unterschiedliche Resistenzquellen zurückzuführen sind, 100 % identisch für die verglichenen ca. 4100 Basenpaare. Außerdem wurden vollständig diagnostische Polymorphismen in der Nukleotidsequenz gefunden, von denen mehrere zu Aminosäuresubstitutionen in der Protein-Sequenz führen (siehe Figur 1, 2 und3). Einige diese Substitutionen besonders in den Domänenbereichen könnten den Funktionsverlust des identifizierten Resistenzproteins in den ss-Genotypen verursachen. Ferner wurden ebenfalls im Promotorbereich drei mit der Resistenz vollständig gekoppelte (Linkage diseqilibrium = 1) INDELs gefunden (Fig 1). Diese INDELs sind auch als potenzielle Kandidaten für den Funktionsverlust zu betrachten.

### Verifizierung des Gens mittels engen Rekombinanten

In der analysierten Population mit 8004 Pflanzen wurden 16 Rekombinaten in der Zielregion identifiziert (feinkartierter Bereich mit 37996 Basenpaaren). Von diesen 16 Genotypen enthielten 9 Pflanzen die Rekombination zwischen dem NB-ARC-LRR-Protein und dem benachbarten Ankyrin repeat-Protein auf der rechte Seite. Im Falle von zwei Pflanzen sind die Rekombinationen zwischen dem NB-ARC-LRR- Protein und dem links benachbarten DUF565 Protein (Protein mit unbekannter Funktion). Durch die Analyse der Nachkommenschaft von allen diesen rekombinanten Pflanzen (ein Gen Entfernung links und rechts) konnte ganz eindeutig bewiesen werden, dass das Gen zwischen DUF565 und das Ankyrin repeat Protein liegt, nämlich dass nur das NB-ARC Protein für die Resistenz verantwortlich ist.

### Exemplarischer Nachweis der Abwesenheit der Transposoninsertion

Für den Nachweis der Retrotransposon-Insertion wurden 3 spezielle dominante Primerkombinationen entwickelt. Die erste und die zweite Primerkombinationen sind in der Lage die Insertion zu detektieren, da jeweils ein Primer von den beiden Primerpaaren in dem Retrotransposon sitzen (linke oder rechte Flanke des Retrotransposons) und der zweite Primer bindet direkt vor oder nach dem Retrotransposon. Ein drittes Primerpaar detektiert das Fehlen des Retrotransposons, dadurch, dass die Primer vor und nach dem Retrotransposon eine Bindungsstelle finden. Ein PCR-Produkt unter Standardbedingungen kann nur dann entstehen, wenn das Retrotransposon fehlt, ansonsten mit dem Retrotransposon wäre das PCR Produkt zu groß und so würde auf diese Weise kein Amplikon entstehen.

### Verifizierung des Gens mittels RNAi-Ansatz

Neben der oben beschriebenen_Verifizierung des Gens mittels engen Rekombinanten erfolgte auch ein weiterer Nachweis der Resistenzwirkung des Gens mittels RNA-Interferenz. Hierfür wurde ein resistenter Standard-Zuckerrübengenotyp mit einem DNA-Konstrukt transformiert, welches eine doppelsträngige hairpin-RNA kodiert. Diese dsRNA war in der Lage posttransskriptional ein Gene Silencing zu bewirken, welches das resistente RZ-3-Genallel in seiner Wirkung vermindern oder ausschalten würde, wodurch der zuvor resistente Zuckerrübengenotyp sensitiv gegenüber Rizomania werden sollte.

Zur Bereitstellung eines geeigneten DNA-Konstrukts wurde ein definierter Zielsequenzbereich des resistenten RZ3-Genallels von 434 Basenpaaren Länge (SEQ ID NO: 7; Figur 4) ausgewählt, durch PCR amplifiziert und sowohl in sense- als auch in antisense-Richtung in den Vektor pZFN kloniert, der zur Synthese von hairpin-Strukturen geeignet ist (Fig. 6). Dieser Vektor weist einen doppelten CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen nos-Terminator auf. Die Transformation der Zuckerrüben mit dem bereitgestellten Vektor erfolgte nach dem Protokoll von Lindsey & Gallois (1990) unter Verwendung des Antibiotikums Kanamycin als Selektionsmarker. Nach mehreren Selektionsschritten wurde die erfolgreiche Transformation an transgenen Sprosse über PCR mittels Nachweis der Anwesenheit des nptll-Gen, des AAP6-Intron und der beiden t-DNA-Bordersequenzen (LB/RB) und der Abwesenheit von *vir* überprüft. Positive Sprosse wurden *invitro* auf jeweils 30 Sprosse klonal vermehrt, bewurzelt und im Gewächshaus in Erde überführt. Etwa 2 Wochen später wurden die transgenen Zuckerrübenpflanzen in Rizomaniakontaminierte Erde pikiert, in welcher sie für 8 bis 10 Wochen kultiviert wurden. Als Kontrolle wurden unter denselben Bedingungen nicht transformierte Pflanzen desselben resistenten genetischen Standard-Transformationshintergrunds herangezogen. Zum Nachweis der Ausprägung der Rizomania wurden die Wurzeln der Zuckerrübenpflanzen geerntet und mittels ELISA-Test der BNYVV-Befall quantifiziert, wobei ein niedriger ELISA-Wert eine Resistenz und ein hoher Wert eine Sensitivität anzeigt (Mechelke 1997, Clark & Adams 1977). Der ELISA-Wert der transformierten Zuckerrüben war mit einem Mittelwert von 3,55 signifikant höher als der ELISA-Wert der weiterhin resistenten Kontrolle mit einem Mittelwert von 1,27 und vergleichbar mit dem sensitiven Standard D108_ss (Tabelle 1). Danach zeigten die Ergebnisse des ELISA-Tests, dass durch das spezifische Gene-Silencing des resistenten RZ-3-Allels im Transformationshintergrund eine zuvor resistente Pflanze sensitiv gegenüber BNYVV wurde. Folglich konnte das Gen der vorliegenden Erfindung eindeutig als das Resistenzgen RZ3 verifiziert werden.

**Tabelle 1: Ergebnisse des ELISA-Tests nach statistischer Analysen (D108_ss = sensitiver Standard; 6921_RR = resistenter Transformationshintergrund; 6921_RNAi = resistenter Transformationshintergrund mit dsRNA gerichtet gegen RZ3-Gen).**

| | D108_ss | 6921_RR | 6921_RNAi |
|---|---|---|---|
| n | 6 | 25 | 64 |
| Mittelwert | 3,98 | 1,27 | 3,55 |
| Standardfehler | 0,02 | 0,25 | 0,11 |
| Standardabweichung | 0,06 | 1,24 | 0,87 |
| *T-Test (Signifikanzlevel): p* < *0,0001* | | | |

### Referenzen

Clark, M.F.; Adams, A.N. (1977): Characteristics of the microplate method of enzyme-linked immunosorbent assay for the detection of plant viruses. J. Gen. Virol. 34, 475-483
Depicker A, Stachel S, Dhaese P, Zambryski P, Goodman HM (1982) Nopaline synthase: transcript mapping and DNA sequence. J Mol Appl Genet. 1(6): 561-73.
Esser K (2000) Kryptogamen 1: Cyanobakterien Algen Pilze Flechten Praktikum und Lehrbuch. Springer-Verlag, Berlin, Heidelberg, 3. Aufl. 2000.
Gidner S, Lennefors BL, Nilsson NO, Bensefelt J, Johansson E, Gyllenspetz U, Kraft T (2005) QTL mapping of BNYVV resistance from the WB41 source in sugar beet. Genome 48: 279-285.
Larson RL, Wintermantel WM, Hill A, Fortis L, Nunez A (2008) Proteome changes in sugar beet in response to Beet necrotic yellow vein virus. Physiological and Mol. PI. Pathol. 72: 62-72.
Lindsey, K., and P. Gallois (1990) "Transformation of sugarbeet (Beta vulgaris) by Agrobacterium tumefaciens." Journal of experimental botany 41.5: 529-536.
Martin GB, Bogdanove AJ; Sessa G (2003) Understanding the functions of plant disease resistance proteins. Annual Review of Plant Biology 54: 23-61.
Mechelke W (1997) Probleme in der Rizomaniaresistenzzüchtung, Vorträge für Pflanzenzüchtung, Resistenzzüchtung bei Zuckerrüben, Gesellschaft für Pflanzenzüchtung e.V., 113-123.
Odell JT, Nagy F, Chua N-H (1985) Identification of DNA sequences required for activity of the cauliflower mosaic virus 35S promoter. Nature 313, 810 - 812
Rushton PJ, Torres JT, Parniske M, Wernert P, Hahlbrock K, and Somssich IE (1996) Interaction of elicitor-induced DNA-binding proteins with elicitor response elements in the promoters of parsley PR1 genes. EMBO J. 15(20): 5690-5700.
Sambrook J, Russell DW (2001) Molecular Cloning. A laboratory manual, Cold Spring Harbor Laboratory Press, 3. Aufl. 2001.
Schmidlin LEDEB, Weyens G, Lefebvre M, Gilmer D (2008) Identification of differentially expressed root genes upon rhizomania disease. Mol. Plant Pathol. 9(6):741-51.
Scholten OE, Bock TSMD, Klein-Lankhorst RM, Lange W (1999) Inheritance of resistance to Beet necrotic yellow vein virus in Beta vulgaris conferred by a second gene for resistance. Theor. Appl. Genet. 99:740-746.
Sohi HH, Maleki M(2004) Evidence for presence of types A and B of beet necrotic yellow vein virus (BNYVV) in Iran. Virus Genes 29(3): 353-8.
Van Ooijen G, Mayr G, Kasiem MMA, Albrecht M, Cornelissen BJC, Takken FLW (2008) Structure-function analysis of the NB-ARC domain of plant disease resistance proteins. Journal of Experimental Botany, 59(6): 1383-1397
WO/2000/29592 (Max-Planck-Gesellschaft zur Förderung der Wissenschaften e.V.). Chimeric promoters capable of mediating gene expression in plants upon pathogen infection and uses thereof.
WO/2006/128444 (KWS SAAT AG). AUTOACTIVATED RESISTANCE PROTEIN.
WO/2007/147395 (KWS SAAT AG). Pathogen induzierbarer synthetischer Promotor.
WO/2013/127379 (KWS SAAT AG). PATHOGEN-RESISTANT TRANSGENIC PLANT.
WO/2013/050024 (KWS SAAT AG). TRANSGENIC PLANT OF THE SPECIES BETA VULGARIS HAVING ENHANCED RESISTANCE TO CERCOSPORA.
WO/2013/091612 (KWS SAAT AG). NOVEL PLANT-DERIVED CIS-REGULATORY ELEMENTS FOR THE DEVELOPMENT OF PATHOGEN-RESPONSIVE CHIMERIC PROMOTORS.

## Patentansprüche

1. BNYVV-resistente transgene Rote Bete Pflanzenzelle der Art *beta vulgaris* ssp. *vulgaris* var. *conditiva,* umfassend als Transgen ein Nukleinsäuremolekül, das eine Nukleotidsequenz umfasst, welche ausgewählt ist aus
a) einer Nukleotidsequenz, die ein Polypeptid mit einer Aminosäuresequenz gemäß SEQ ID NO: 2 oder SEQ ID NO: 3 kodiert,
b) einer Nukleotidsequenz, die die kodierende Sequenz der DNA-Sequenz gemäß SEQ ID NO: 1 umfasst,
c) einer Nukleotidsequenz, die ein Polypeptid kodiert, welches sich durch Substitution, Deletion und/oder Addition von einer Aminosäure der Aminosäuresequenz, die durch die Nukleotidsequenz nach a) oder b) kodiert wird, von einem Polypeptid, das durch die Nukleotidsequenz nach a) oder b) kodiert wird, ableitet,
d) einer Nukleotidsequenz, die ein Polypeptid kodiert, welches eine Aminosäuresequenz aufweist, die zu mindestens 80% identisch ist zu einer Aminosäuresequenz, welche durch die Nukleotidsequenz nach a) oder b) kodiert wird, oder
e) einer Nukleotidsequenz, welche mindestens die Aminosäurepositionen 168-227 der SEQ ID NO: 2 und mindestens die Aminosäurepositionen 591-613 der SEQ ID NO: 2 und mindestens die Aminosäurepositionen 1013-1072 der SEQ ID NO: 2 oder welche mindestens die Aminosäurepositionen 182-241 der SEQ ID NO: 3, mindestens die Aminosäurepositionen 605-627 der SEQ ID NO: 3 und mindestens die Aminosäurepositionen 1027-1086 der SEQ ID NO: 3 kodiert.

2. Pflanze oder Teil davon, umfassend eine Pflanzenzelle nach Anspruch 1, **dadurch gekennzeichnet, dass** die Pflanze oder der Teil davon Resistenz gegen BNYVV aufweist, wobei die Pflanze das von dem Nukleinsäuremolekül, wie definiert in Anspruch 1, kodierte Polypeptid nach Expression umfasst.

3. Samen einer Pflanze nach Anspruch 2, wobei der Samen mindestens eine Pflanzenzelle wie definiert in Anspruch 1 umfasst.

4. Verfahren zur Selektion einer BNYVV-resistenten Rote Bete Pflanzenzelle oder Pflanze der Art *beta vulgaris* ssp. *vulgaris* var. *conditiva,* durch Nachweisen eines Nukleinsäuremoleküls umfassend eine Nukleotidsequenz, wie definiert in Anspruch 1, in einer Rote Bete Pflanzenzelle der Art *beta vulgaris* ssp. *vulgaris* var. *conditiva,* wobei das Verfahren umfasst:
(i.) Nachweisen der Abwesenheit einer Insertion in der kodierenden Nukleotidsequenz des Nukleinsäuremoleküls wie definiert in Anspruch 1; oder
(ii.) Nachweisen von mindestens einem Polymorphismus gemäß Figur 1, 2 und/oder 3 in der kodierenden Nukleotidsequenz des Nukleinsäuremoleküls wie definiert in Anspruch 1 unter Verwendung von molekularen Markern, welche den Polymorphismus erkennen; und
(iii.) Selektion einer BNYVV-resistenten Rote Bete Pflanzenzelle oder Pflanze der Art *beta vulgaris* ssp. *vulgaris* var. *conditiva.*

## Claims

1. A BNYVV-resistant transgenic beet plant cell of the species *Beta vulgaris* ssp. *vulgaris* var. *conditiva* comprising as transgene a nucleic acid molecule comprising a nucleotide sequence selected from
a) a nucleotide sequence encoding a polypeptide having an amino acid sequence according to SEQ ID NO: 2 or SEQ ID NO: 3,
b) a nucleotide sequence comprising the coding sequence of the DNA sequence according to SEQ ID NO: 1,
c) a nucleotide sequence encoding a polypeptide, which is derived from a polypeptide encoded by the nucleotide sequence according to a) or b) by substitution, deletion and/or addition of an amino acid of the amino acid sequence encoded by the nucleotide sequence according to a) or b),
d) a nucleotide sequence encoding a polypeptide having an amino acid sequence, which is at least 80% identical to an amino acid sequence encoded by the nucleotide sequence according to a) or b), or
e) a nucleotide sequence, which encodes at least amino acid positions 168-227 of SEQ ID NO: 2 and at least amino acid positions 591-613 of SEQ ID NO: 2 and at least amino acid positions 1013-1072 of SEQ ID NO: 2 or which encodes at least amino acid positions 182-241 of SEQ ID NO: 3, at least amino acid positions 605-627 of SEQ ID NO: 3 and at least amino acid positions 1027-1086 of SEQ ID NO: 3.

2. A plant or part thereof comprising a plant cell as defined in claim 1, **characterized in that** the plant or part thereof has resistance to BNYVV, wherein the plant comprises the polypeptide encoded by the nucleic acid molecule as defined in claim 1 after expression.

3. A seed of a plant as defined in claim 2, wherein the seed comprises at least one plant cell as defined in claim 1.

4. A method for selecting a BNYVV-resistant beet plant cell or plant of the species *Beta vulgaris* ssp. *vulgaris* var. *conditiva,* by detecting a nucleic acid molecule comprising a nucleotide sequence as defined in claim 1 in a beet plant cell of the species *Beta vulgaris* ssp. *vulgaris* var. *conditiva,* wherein the method comprises:
(i.) detecting the absence of an insertion in the coding nucleotide sequence of the nucleic acid molecule as defined in claim 1; or
(ii.) detecting at least one polymorphism according to Figures 1, 2 and/or 3 in the coding nucleotide sequence of the nucleic acid molecule as defined in claim 1, using molecular markers that recognize the polymorphism; and
(iii.) selecting a BNYVV-resistant beet plant cell or plant of the species *Beta vulgaris* ssp. *vulgaris* var. *conditiva.*

## Revendications

1. Cellule végétale de betterave potagère transgénique résistante au BNYVV, de l'espèce *beta vulgaris* ssp. *vulgaris* var. *conditiva,* comprenant comme transgène une molécule d'acide nucléique qui comprend une séquence nucléotidique qui est choisie parmi
a) une séquence nucléotidique qui code pour un polypeptide ayant une séquence d'acides aminés selon SEQ ID NO : 2 ou SEQ ID NO : 3,
b) une séquence nucléotidique qui comprend la séquence codante de la séquence d'ADN selon SEQ ID NO : 1,
c) une séquence nucléotidique qui code pour un polypeptide qui est dérivé par substitution, délétion et/ou addition d'un acide aminé de la séquence d'acides aminés qui est codée par la séquence nucléotidique selon a) ou b), d'un polypeptide qui est codé par la séquence nucléotidique selon a) ou b),
d) une séquence nucléotidique qui code pour un polypeptide qui possède une séquence d'acides aminés qui est au moins à 80 % identique à une séquence d'acides aminés qui est codée par la séquence nucléotidique selon a) ou b), ou
e) une séquence nucléotidique qui code au moins les positions d'acides aminés 168 à 227 de SEQ ID NO : 2 et au moins les positions d'acides aminés 591 à 613 de SEQ ID NO : 2 et au moins les positions d'acides aminés 1013 à 1072 de SEQ ID NO : 2 ou qui code au moins les positions d'acides aminés 182 à 241 de SEQ ID NO : 3, au moins les positions d'acides aminés 605 à 627 de SEQ ID NO : 3 et au moins les positions d'acides aminés 1027 à 1086 de SEQ ID NO : 3.

2. Plante ou partie de plante, comprenant une cellule végétale selon la revendication 1, **caractérisée par le fait que** la plante ou la partie de plante présente une résistance au BNYVV, dans laquelle la plante comprend le polypeptide codé par la molécule d'acide nucléique telle que définie dans la revendication 1, après expression.

3. Semence d'une plante selon la revendication 2, dans laquelle la semence comprend au moins une cellule végétale telle que définie dans la revendication 1.

4. Procédé de sélection d'une cellule végétale ou plante de betterave potagère résistante au BNYVV, de l'espèce *beta vulgaris* ssp. *vulgaris* var. *conditiva,* par détection d'une molécule d'acide nucléique comprenant une séquence nucléotidique telle que définie dans la revendication 1, dans une cellule végétale de betterave potagère de l'espèce *beta vulgaris* ssp. *vulgaris* var. *conditiva,* le procédé comprenant :
(i.) détecter l'absence d'une insertion dans la séquence nucléotidique codante de la molécule d'acide nucléique telle que définie dans la revendication 1 ; ou
(ii.) détecter au moins un polymorphisme selon les figures 1, 2 et/ou 3 dans la séquence nucléotidique codante de la molécule d'acide nucléique telle que définie dans la revendication 1, en utilisant des marqueurs moléculaires qui reconnaissent le polymorphisme ; et
(iii.) sélectionner une cellule végétale ou plante de betterave potagère résistante au BNYVV, de l'espèce *beta vulgaris* ssp. *vulgaris* var. *conditiva.*
